# EUROPEAN PATENT APPLICATION

(11) **EP 1 566 436 A1**
(43) Date of publication of application: **24.08.2005**
(21) Application number: 03774110.5
(22) Date of filing: 20.11.2003
(51) Int. Cl.: C12N 15/10, C12N 15/63, C12Q 1/02

(54) **A METHOD FOR ISOLATING A NUCLEIC ACID HAVING AN INTENDED FUNCTIONAL PROPERTY ANDA KIT THEREFOR**

(30) Priority: 21.11.2002 JP 2002337545
(71) Applicant: Genomidea Inc, Osaka 530-0043 (JP)
(72) Inventor: KANEDA, Yasufumi, Mino-shi, Osaka 562-0031 (JP); NISHIKAWA, Tomoyuki, Mino-shi, Osaka 562-0031 (JP)
(74) Representative: Harding, Charles Thomas
(86) International application number: PCT/JP2003/014857
(87) International publication number: WO 2004/046353

(57) **Abstract**

The present invention provides a method for isolating a nucleic acid having an intended functional property conveniently and rapidly, as well as a kit for carrying out the method. Specifically, the present invention provides a method comprising the steps of: (A) transferring a nucleic acid into a plurality of first host cells and allowing the nucleic acid to transiently express therein; (B) selecting, from the first host cells into which the nucleic acid is transferred, a cell which a nucleic acid having an intended functional property has been transferred; (C) preparing a purified nucleic acid from the selected cell; and (D) selecting a purified nucleic acid having an intended functional property, as well as a kit to carry out the method.

## Description

### TECHNICAL FIELD

The present invention relates to a method of isolating a nucleic acid having an intended functional property and a kit for carrying out the method.

### BACKGROUND ART

Expression cloning is a method of cloning a gene that uses a function exhibited as a result of expression of the target gene as an index. This method does not requires information such as the base sequence of the gene or the amino acid sequence of the gene product, and is advantageous when cloning genes whose expression amount is small, and/or genes for which only functional information is available.

The gene products of mammals include many proteins that interact with other factors (such as proteins) in mammalian cells. Therefore, when conducting expression cloning of genes from mammalian cells, it is preferable to use the mammalian cells from which the gene is derived.

In the case where expression cloning is conducted using a mammalian cell according to conventional methods, even when a mammalian cell containing nucleic acid having the intended functional property is isolated, usually the isolated mammalian cell simultaneously contains plural kinds of nucleic acids in a single cell, in contrast to prokaryotes and fungi. For this reason, it is necessary to purify the nucleic acid having an intended functional property from the plurality of nucleic acids existing in the isolated cell.

When conventional methods are used, a large amount of labor is required in order to purify a nucleic acid having an intended functional property from an isolated cell. This may be attributed to the following fact; during expression screening using mammalian cells, a known retrovirus or adenovirus is used as a vector system to stably carry a foreign gene, however, these virus vector systems incorporate several vector genes into the chromosome of a host cell, thus in order to purify and identify the transferred nucleic acid the purification process has to be repeated several times. In order to obtain a purified clone, a typical purification process comprises the steps of:
1) PCR amplification of the nucleic acid sequence;
2) transfection of the host cell with the amplified PCR fragment; and
3) selection of transfected cells based on a desired phenotype, and the series of the steps should be repeated 10 to 20 times

Methods of cloning and purifying a mixture of nucleic acids using microorganisms such as Escherichia coli are well known. Theoretically, after isolating a first host cell containing plural kinds of nucleic acids, a specific nucleic acid may be purified from the plural kinds of transferred nucleic acids using a second host cell such as Escherichia coli. Such purification methods require, for example, the steps of amplifying a nucleic acid incorporated into a chromosome by PCR or the like; ligating the amplified nucleic acid into a vector that autonomously replicates in a second host cell; and transferring the ligated product into a second host cell. However, this ligation step is very inefficient, and as the number of individual nucleic acids to be ligated increases, the chances of succeeding in purifying the target nucleic acid reduces. For this reason, in conventional methods using retroviruses, adenoviruses and the like, a second host cell such as Escherichia coli is not used for the purpose of purifying a specific nucleic acid, for example, during expression cloning.

As a means for transferring a foreign nucleic acid into a mammalian cell, besides the method of using viruses such as retroviruses, conventional methods such as calcium precipitation are also well-known. However, when transient expression is conducted using conventional methods such as calcium precipitation, unlike methods using retroviruses, adenoviruses or the like, degradation of the nucleic acid occurs during transduction, and the nucleic acid fragment encoding the transferred foreign gene is unstable in the host cell. These problems are due to an intrinsic complication of conventionally used methodsof transient expression, and thus transient expression systems such as calcium precipitation are thought to be unsuited as means for obtaining a clone.

Eventually, in conventional methods, even if a candidate nucleic acid is isolated, the candidate gene is usually a population of clones including a plurality of different clones, thus it is necessary to purify and isolate a single clone from the population of clones. This purification requires a large amount of labor in terms of multiple screening steps, and this obstructs practical isolation and screening.

Therefore, it is an object of the present invention to provide a novel method of simply and rapidly isolating a nucleic acid (e.g., screening method), and to provide a kit for carrying out the method.

### SUMMARY OF THE INVENTION

Therefore, the present invention provides a novel method of isolating a nucleic acid conveniently and rapidly (e.g., screening method), as well as a kit for carrying out the method. Byusing the method and kit of the present invention, it is possible to carry out screening, particularly expression screening using mammalian cells, rapidly and conveniently. Conventionally, during expression screening usingmammalian cells, a large amount of labor and time was required in order to purify a candidate nucleic acid, however the required labor and time can largely be reduced using the present invention. Therefore, the effect of the present invention is significant.

The present invention has the following features:
(1) A method of isolating a nucleic acid having an intended functional property, comprising the steps of:
   (A) transferring a nucleic acid into a plurality of first host cells and allowing the nucleic acid to transiently express therein;
   (B) selecting, from the plurality of first host cells into which the nucleic acid is transferred, a cell into which a nucleic acid having an intended functional property has been transferred;
   (C) preparing a purified nucleic acid from the selected cell; and
   (D) selecting a purified nucleic acid having an intended functional property.
(2) The method according to item (1), wherein at least two kinds of nucleic acids are transferred into the plurality of first host cells.
(3) The method according to item (1), wherein the step of transferring a nucleic acid into the plurality of first host cells is carried out according to a procedure selected from the group consisting of: a transferring method using a viral envelope, a transferring method using a liposome, a transferring method using a liposome containing at least one protein from a viral envelope, a transferring method using calcium phosphate and an electroporation method.
(4) The method according to item (1), wherein the nucleic acid includes a foreign gene and a promoter.
(5) The method according to item (1), wherein the host cells are mammalian cells.
(6) The method according to item (1), wherein the host cells are human cells.
(7) The method according to item (1), wherein the viral envelope is derived from wild-type or recombinant viruses.
(8) The method according to item (1), wherein the viral envelope is derived from a virus belonging to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae.
(9) The method according to item (8), wherein the virus is derived from viruses belonging to the family Paramyxoviridae.
(10) The method according to item (9), wherein the virus is HVJ.
(11) The method according to item (1), wherein the vector is a viral envelope vector.
(12) The method according to item (1), wherein the vector is a vector containing a protein prepared from a viral envelope and a liposome.
(13) The method according to item (12), wherein the protein prepared from a viral envelope is a protein selected from the group consisting of F protein, HN protein, NP protein and a combination thereof.
(14) The method according to item (1), wherein the step (C) of preparing a purified nucleic acid from the selected cell is carried out in the following steps of:
   (i) extracting a nucleic acid from the selected cell;
   (ii) transferring the extracted nucleic acid into a second host cell to thereby obtain a transformed cell;
   (iii) purifying the transformed cell; and
   (iv) preparing a nucleic acid from the purified transformed cell.
(15) The method according to item (14), wherein the second host cell is a bacterium or a fungus.
(16) The method according to item (15), wherein the nucleic acid contains a sequence that is necessary for autonomous replication in the bacterium or fungus.
(17) The method according to item (15), wherein the bacterium belongs to a genus selected from the group consisting of Escherichia, Bacillus, Streptococcus, Staphylococcus, Haemophilus, Neisseria, Actinobacillus and Acinetobacter.
(18) The method according to item (17), wherein the bacterium is Escherichia coli.
(19) The method according to item (15), wherein the fungus is Saccharomyces, Schizosaccharomyces or Neurospora.
(20) The method according to item (1), wherein the step (D) of selecting a purified nucleic acid having an intended functional property is carried out in the following steps of:
   (i) transferring the purified nucleic acid into a third host cell to obtain a transformed cell;
   (ii) comparing the property of the transformed cell with the property of a third host cell that is not transformed; and
   (iii) determining whether or not the transformed cell has an intended functional property, as a result of the comparison.
(21) The method according to item (20), wherein the step (D) of selecting a purified nucleic acid having an intended functional property further includes the step of (iv) preparing a nucleic acid having an intended functional property from the selected cell.
(22) The method according to item (20), wherein the third host cell is a mammalian cell.
(23) The method according to item (20), wherein the third host cell is a human cell.
(24) The method according to item (20), wherein the third host cell is derived from the same species as the species from which the first host cell is derived.
(25) The method according to item (1), wherein the intended functional property is selected from the group consisting of induction of angiogenesis, tumor suppression, enhancement of osteogenesis, induction of apoptosis, cytokine secretion, induction of dendrites, suppression of arteriosclerosis, suppression of diabetes; suppression of autoimmune diseases; suppression of Alzheimer's disease, suppression of Parkinson's disease, protection of nerve cells and combinations thereof.
(26) A kit for isolating a nucleic acid having an intended functional property, comprising:
   (A) a nucleic acid transfer vector to be transferred into a plurality of the first host cells in order to transform said first host cells; and
   (B) a second host cell for preparing a purified nucleic acid from a cell selected from the transformed first host cells.
(27) The kit according to item (26), wherein the nucleic acid transfer vector is a viral envelope, liposome or liposome containing at least one protein from viral envelope.
(28) The kit according to item (26), wherein the first host cells are mammalian cells.
(29) The kit according to item (26), wherein the first host cells are human cells.
(30) The kit according to item (26), wherein the viral envelope is derived from wild-type or recombinant viruses.
(31) The kit according to item (26), wherein the viral envelope is derived from a virus belonging to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae.
(32) The kit according to item (26), wherein the virus is derived from viruses belonging to the family Paramyxoviridae.
(33) The kit according to item (26), wherein the virus is HVJ.
(34) The kit according to item (26), wherein the vector is a viral envelope vector.
(35) The kit according to item (26), wherein the vector is a vector containing a protein prepared from a viral envelope and a liposome.
(36) The kit according to item (35), wherein the protein prepared from a viral envelope is a protein selected from the group consisting of F protein, HN protein, NP protein and a combination thereof.
(37) The kit according to item (26), wherein the second host cell is a bacterium or fungus.
(38) The kit according to item (26), further comprising a nucleic acid for preparing a nucleic acid to be transferred into the first host cells.
(39) The kit according to item (26), further comprising a reagent to be used for determining whether or not the purified nucleic acid has an intended functional property.
(40) The kit according to item (37), wherein the bacterium belongs to a genus selected from the group consisting of Escherichia, Bacillus, Streptococcus, Staphylococcus, Haemophilus, Neisseria, Actinobacillus and Acinetobacter.
(41) The kit according to item (40), wherein the bacterium is Escherichia coli.
(42) The kit according to item (37), wherein the fungus is Saccharomyces, Schizosaccharomyces or Neurospora.
(43) A nucleic acid isolated by the method according to item (1).
(44) Use of a viral envelope for isolating a nucleic acid having an intended functional property.
(45) Use of a liposome containing at least one protein from a viral envelope, for isolating a nucleic acid having an intended functional property.

### BRIEF DESCRIPTION OF THE DRAWINGS

Fig. 1 is a schematic showing the steps in genome screening using HVJ-E.
Fig. 2 shows the result of an HAEC cell growth assay after transferring a genomic library gene.
Fig. 3 is a computer-generated graph, showing the cell growth states of each well in a human heart pcDNA library screening.
Fig. 4 is a schematic showing confirmation of an insert by digesting cloned genes using restriction enzymes.
Fig. 5 shows the result of a second cell growth assay.
Fig. 6 shows micrographs of each well at 40-fold magnification.
Fig. 7 is a graph comparing areas of cells exhibiting angiogenesis (left) and a graph comparing the length of cells exhibiting angiogenesis (right) obtained by using an angiogenesis quantification software.
Fig. 8 is a graph comparing joint numbers of the junctions of cells exhibiting angiogenesis (left) and a graph comparing the numbers of paths of cells exhibiting angiogenesis (right).
Fig. 9 is a graph comparing the effect of the clone on c-fos gene promoter activity.

### DETAILED DESCRIPTION OF THE INVENTION

Throughout the following specification explaining the present invention, it is to be understood that articles for singular forms (e.g., "a", "an", "the" in English, "ein", "der", "das", "die" and their declined forms in German, "un", "une", "le", "la" in French, and "un", "una", "el", "la" in Spanish, and corresponding articles and adjectives in other languages) also imply concepts of plural forms, unless otherwise indicated. In addition, the terms used in this specification should be understood as being used in the sense that is generally used in the art, unless otherwise indicated.

### (DEFINITION)

The term "cell" as used herein is defined in a similar manner to the broadest meaning used in the art, and refers to a living organism which is a sub-unit of tissue of a multicellular organism, encapsulated by a membrane structure that separates it from the external environment, is self-reproducing and carries genetic information and an expression system therefore.

The terms "protein", "polypeptide" and "peptide", as used herein may be interchangeably used, and each term refers to a macromolecule (polymer) comprising a sequence of amino acids. The term "amino acid" refers to an organic molecule having a carboxylic group and an amino group at a carbon atom. Preferably, the amino acids used in this specification are usually, but are not limited to, the 20 naturally occurring amino acids.

The terms "nucleic acid", "nucleic acid molecule", "polynucleotide" and "oligonucleotide" as used herein are interchangeably used unless otherwise indicated, and each term refers to a macromolecule (polymer) comprising a sequence of nucleotides. The term "nucleotide" refers to a nucleoside in which the 5' moiety of ribose is a phosphate ester. Nucleotides having a pyrimidine base or purine base (pyrimidine nucleotide and purine nucleotide) as a base moiety are known. A polynucleotide includes deoxyribonucleic acid (DNA) or ribonucleic acid (RNA).

This term also includes "derivative oligonucleotide" or "derivative polynucleotide". The term "derivative oligonucleotide" or "derivative polynucleotide" is interchangeably used, and refers to an oligonucleotide or polynucleotide which contains a derivative of a nucleotide or an oligonucleotide in which the bond between two or more nucleotides is not the normal one.

Concrete examples of such oligonucleotides include: 2'-O-methyl-ribonucleotide; a derivative oligonucleotide in which a phosphodiester bond in the oligonucleotide is changed toaphosphorothioatebond; a derivative oligonucleotide in which a phosphodiester bond in the oligonucleotide is changed to a N3'-P5' phosphoroamidate bond; a derivative oligonucleotide in which a ribose and phosphodiester bond in the oligonucleotide is changed into a peptide-nucleic acid bond; a derivative oligonucleotide in which a uracil in the oligonucleotide is substituted by C-5 propynyluracil; a derivative oligonucleotide in which a uracil in the oligonucleotide is substituted by C-5 thiazole uracil; a derivative oligonucleotide inwhich a cytosine in the oligonucleotide is substituted by C-5 propynyl cytosine; a derivative oligonucleotide in which a cytosine in the oligonucleotide is substituted by phenoxazine-modified cytosine; a derivative oligonucleotide in which a ribose moiety in a DNA molecule is substituted by 2'-O- propyl ribose; and a derivative oligonucleotide in which a ribose moiety in the oligonucleotide is substituted by 2'-methoxyethoxy ribose. Unless otherwise specified, a specific nucleic acid sequence is intended to encompass conservative variants (for example, degenerate codon substitutes) or complementary sequences thereof as well as the specific sequence given. Specifically, a degenerate codon substitute can be achieved by creating a sequence in which the third position of one or more selected (or all) codon(s) is substituted by a mixed base and/or a deoxyinosine residue (Batzer et al., Nucleic Acid Res. 19:5081(1991); Otsuka et al., J. Biol. Chem. 260: 2605-2608 (1985); Rossolini et al., Mol. Cell. Probes 8: 91-98 (1994)). The term "nucleic acid" as used herein is also used interchangeably with gene, cDNA, RNA, mRNA, oligonucleotide and polynucleotide.

The term "gene" as used herein means a factor that defines inherited characteristics. Usually, genes are arranged on a chromosome in a certain order. A gene that defines the primary structure of protein is called a structural gene, and a gene that regulates the expression of a structural gene is called a regulatory gene. In this specification, the term "gene" sometimes refers to a "polynucleotide", "oligonucleotide" and "nucleic acid".

The term "fragment" of a nucleic acid molecule as used herein refers to a polynucleotide that is shorter than the entire length of the reference nucleic acid molecule, but has a length sufficient for use as a factor of the present invention. Therefore, a fragment as used herein is a polypeptide or a polynucleotide having a sequence length of 1 to n-1 relative to the full length of a polypeptide or a polynucleotide (having a length of n). The length of the fragment may be appropriately selected depending on the purpose thereof, and a lower limit of length for a polypeptide can include 3, 4, 5, 6, 7, 8, 9, 10, 15, 20, 25, 30, 40, 50 and more amino acids. Lengths represented by integers not specifically recited above (for example, 11) are also suitable as a lower limit. Polynucleotides can include 5, 6, 7, 8, 9, 10, 15, 20, 25, 30, 40, 50, 75, 100 and more nucleotides. Lengths represented by integers not specifically recited above (for example, 11) are also suitable as a lower limit.

The term "homology" of a gene as used herein represents the degree of identity to each other between two ore more gene sequences. Therefore, the higher the homology of two specific genes, the higher the identity and similarity of their sequences are. Whether or not two selected genes have homology may be examined by a direct comparison of their sequences, or hybridization under stringent conditions in the case of nucleic acid sequences. During direct comparison of two gene sequences, typically, when at least 50%, preferably at least 70%, more preferably, when at least 80%, 90%, 95%, 96%, 97%, 98% or 99% of the DNA sequences are identical between the two gene sequences, the genes are determined to have homology.

Comparison of similarity and identity between base sequences and determination of homology of base sequences are carried out herein by means of BLAST which is a sequence analysis tool using default parameters.

"Expression" of a gene, polynucleotide, polypeptide or the like refers to the phenomenon that the gene or the like takes a different form under certain circumstances in vivo. Preferably, it means the phenomena of a gene, polynucleotide or the like being transcribed and translated into a polypeptide. The phenomena of mRNA being produced as a result of transcription may also be one form of expression. More preferably, the resultant polypeptide may have undergone post-translational processing.

The term "a polynucleotide that hybridizes under stringent conditions" as used herein refers to well-known conditions that are commonly used in the art. Such a polynucleotide can be obtained by the colony hybridization method, the plaque hybridization method or Southern blot hybridization using a polynucleotide selected from the polynucleotides of the present invention as a probe. Specifically, it means a polynucleotide that can be identified in the following manner. A filter on which DNA derived from colonies or plaques is immobilized is used to carry out a polynucleotide hybridization in the presence of 0.7-1.0 M NaCl at 65°C. Then, the filter is washed at 65°C with x0.1 to x2 concentration of SSC (saline-sodium citrate) solution (150 mM sodium chloride, 15 mM sodium citrate). Hybridization may be conducted according to methods described in laboratory manuals such as Molecular Cloning 2nd ed. , Current Protocols in Molecular Biology, Supplement 1-38, DNA Cloning 1: Core Techniques, A Practical Approach, Second Edition, Oxford University Press (1995) and the like. Herein, "a sequence that hybridizes under stringent conditions" preferably excludes sequences comprising exclusively A or exclusively T.

The wording "hybridizable polynucleotide" refers to a polynucleotide that is able to hybridize with other polynucleotides under the aforementioned hybridization conditions. Specific examples of hybridizable polynucleotides include polynucleotides having at least 60% or higher homology, preferably 80% or higher homology, more preferably 95% or higher homology with a DNA base sequence encoding a polypeptide having the amino acid sequence set forth in the SEQ ID NO:2, 4 or 6. As to the given homology, similarity may be represented by a score, for example, by using the search program BLAST using the algorithm developed by Altschul et al. (J. Mol. Biol. 215, 403-410 (1990)).

Amino acids may be denoted herein by the generally known three-letter coding or the one-letter coding recommended by IUPAC-IUB Biochemical Nomenclature Commission. Likewise, nucleotides may be denoted by the commonly accepted one-letter coding.

"Corresponding" amino acid used herein refers to an amino acids having or expected to have a similar effect in a protein or polypeptide to the specific amino acid in a protein or polypeptide which is the reference for comparison. In an enzyme molecule, in particular, it means an amino acid which is located at a similar position in an active site and similarly contributes to catalytic activity.

The term "nucleotide" as used herein may be naturally occurring or may not be naturally occurring. The term "derivative nucleotide" or "nucleotide analogue" refers to a nucleotide that is different from a naturally occurring nucleotide, but has a function similar to that of the function of the original nucleotide. Such derivative nucleotides and nucleotide analogues are well known in the art. Examples of such derivative nucleotides and nucleotide analogues include, but are not limited to, phosphorothioate, phosphoroamidate, methyl phosphonate, chiral methyl phosphonate, 2-O-methylribonucelotide and peptide-nucleic acid (PNA).

The term"fragment" as used herein refers to a polypeptide or polynucleotide having a sequence length of 1 to n-1 relative to the full length of a polypeptide or polynucleotide (having a length of n). The length of the fragment may be appropriately selected depending on the purpose thereof, and a lower limit of length for a polypeptide includes 3, 4, 5, 6, 7, 8, 9, 10, 15, 20, 25, 30, 40, 50 and more amino acids. Lengths represented by integers not specifically recited above (for example, 11) are also suitable as a lower limit. For polynucleotides, fragment includes polynucleotides of 5, 6, 7, 8, 9, 10, 15, 20, 25, 30, 40, 50, 75, 100 and more in length. Lengths represented by integers not specifically recited above (for example, 11) are also suitable as a lower limit.

The term "isolation (isolated)" as used herein refers to the state of a certain substance or nucleic acid, and that the substance or nucleic acid is in a state that is different from its naturally-occurring state, and that the substance or nucleic acid is not accompanied by at least one substance or nucleic acid that accompanies it in its naturally-occurring state. The term to "concentrate" a nucleic acid as used herein refers to increasing the abundance of a specific nucleic acid compared to its naturally-occurring abundance. Therefore, in a preferred condition, a concentrated nucleic acid or nucleic acid composition contains only a specific nucleic acid.

The term to "purify" a specific substance as used herein refers to making the substance in it's abundantly existing state, and reducing the concentration of substances other than the specified substance to such a degree that they will not influence the function of the specified substance. Therefore, in a preferred condition, a purified substance or substance-containing composition contains only the specific substance.

In this specification the terms "purification (purified)" and "cloning (cloned)" are interchangeably used. The terms "purification (purified)" and "cloning (cloned)" refer to a state of a certain substance or nucleic acid, and refer to making the abundance of the nucleic acid higher, preferably to the level that the substance or nucleic acid is not substantially accompanied by other kinds of substances or nucleic acids. When used in the contexts of "purification" and "cloning" herein, the term "state where substantially no other kinds of substances or nucleic acids accompany" refers to the state where these other kinds of substances or nucleic acids are completely absent, or will not exert any influence on the substance or nucleic acid of interest if present. Therefore, in a more preferred condition, a purified nucleic acid or nucleic acid composition contains only a specific nucleic acid.

The term "purify" a specific substance as used herein refers to making the substance in an abundantly existing state, and reducing the concentration of substances other than the specified substance to such a degree that they will not influence the function of the specified substance.

The term "gene transfer" as used herein refers to transferring a desired naturally-occurring, synthetic or recombinant gene or gene fragment into a target cell in vivo or in vitro in such a manner that the transferred gene maintains its function. A gene or gene fragment transferred in the present invention encompasses DNA or RNA having a specific sequence, or a nucleic acid which is a synthetic analogue thereof. The terms "gene transfer", "transfection" and "transfect" as used herein are interchangeably used.

The terms "gene transfer vector" and "gene vector" as used herein are interchangeably used. The terms "gene transfer vector" and "gene vector" refer to vectors capable of transferring a polynucleotide sequence of interest into a target cell. Examples of a "gene transfer vector" and "gene vector" include, but are not limited to, a "viral envelope vector" and a "liposome vector".

The term "viral envelope vector" as used herein refers to a vector in which a foreign gene is encapsulated in a viral envelope or a vector in which a foreign gene is encapsulated in a component containing a protein derived from a viral envelope. The virus used for preparing a gene transfer vector may be a wild-type virus or a recombinant virus.

In the present invention, examples of the virus used for preparing a viral envelope or a protein derived from a viral envelope include, but are not limited to, viruses belonging to families selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae. Preferably viruses belonging to the family Paramyxoviridae, and more preferably HVJ (Hemagglutinating Virus of Japan, Sendai Virus) are used.

Examples of proteins derived from a viral envelope include, but are not limited to the F protein, HN protein, NP protein and M protein of HVJ.

The term "liposome vector" as used herein refers to a vector wherein a foreign gene is encapsulated in a liposome. Examples of lipids used for preparing a liposome vector include, but are not limited to, neutral phospholipids such as DOPE (dioleoyl phosphatidyl ethanolamine) and phosphatidyl choline; negatively charged phospholipids such as cholesterol, phosphatidyl serine and phosphatidic acid; and positively charged lipids such as DC-cholesterol (dimethylaminoethanecarbamoyl cholesterol) and DOTAP (dioleoyl trimethylammonium propane).

The term "liposome" used herein is one type of lipid bilayer. For example, when a phospholipid such as lecithin is suspended at 50% (by weight) or more in water at a temperature higher than the gel-liquid crystal phase transition temperature which is specific for said phospholipid, a closed vesicle composed of a lipid bilayer membrane encapsulating a water phase is formed. This vesicle is called a liposome. Liposomes are generally classified as multilayered liposomes (MLV: multilamellar vesicle), in which a plurality of bilayer membranes overlap like an onion, and unilamellar liposomes having only one membrane. The latter may also be prepared by making a suspension of phospholipids such as phosphatidyl choline dispersed by intensive stirring with a mixer, followed by an ultrasound treatment.

Liposomes having only one membrane are further classified into small single membrane liposomes (SUV: small unilamellar vesicle) and large single membrane liposomes (LUV: large unilamellar vesicle) according to their diameter. MLVs are prepared by adding water to a lipid thin film and applying mechanical oscillation. SUVs may be prepared by ultrasonication of MLVs or by removal of a surfactant from a mixture of lipid and surfactant by dialysis or the like. Besides the above methods, other well-known methods include (1) a method of preparing LUV by treating SUV with multiple freeze- thaw cycles; (2) a method of preparing LUVs by fusing SUVs composed of acidic phospholipids in the presence of Ca²⁺ and then removing the Ca²⁺ with EDTA (ethylenediamine tetraacetic acid), and (3) a method of preparing LUVs and the like by causing phase conversion while distilling off ether from an emulsion of lipids in solution with ether and water (reverse-phase evaporation vesicle: REV).

The terms "surfactant" and "surface activator" as used herein are interchangeably used. A surfactant is a substance that exhibits strong surface-tension activity against water, and forms an aggregate such as micelle in a solution at concentrations exceeding the critical micelle concentration. A surfactant has both a hydrophilic moiety and a hydrophobic (lipophilic) moiety , and strongly absorbs into a two-phase interface of water and oil according to the balance of hydrophilicity and lipophilicity, to significantly decrease the free energy (interface tension) at the interface. Typical hydrophobic groups are long chain hydrocarbon groups such as alkyl groups; typical hydrophilic groups may include ionic dissociation groups and nonionic polar groups such as a hydroxyl group. Since surfactants having a carboxyl group, sulfo group, hydrogen sulfate group, and -OSO-OH dissociate in water to become anions, they may be called anionic surfactants. Typical examples include, but are not limited to, fatty acid soaps, alkyl benzene sulfonate and the like. In contrast, those having a quaternary ammonium group dissociate to become cations and are called cationic surfactants. There are also surfactants having both a cationic dissociation group and an anionic dissociation group in the same molecule, such as long chain alkyl amino acids, and such surfactants are called amphoteric surfactants. Those surfactants having a nonionic polar group are called nonionic surfactants, polyoxyethylenenonylphenyl ether is typical example of a non-ionic surfactant.

The term "lipid" as used herein encompasses any lipids, as long as (1) they have a long chain fatty acid or a similar hydrocarbon chain in the molecule, and (2) they exist in an organism or they are molecules derived from an organism. Preferred lipids are phospholipids capable of forming liposomes, and more preferred lipids include, but are not limited to, phosphatidyl choline, phosphatidyl serine, phosphatidyl inositol, phosphatidyl ethanol amine, cholesterol, sphingomyelin and phosphatidic acid.

The term "fatty acid" as used herein refers to aliphatic monocarboxylic acids and aliphatic dicarboxylic acids that are obtained by hydrolysis of naturally-occurring lipids. Typical fatty acids include, but are not limited to, arachidonic acid, palmitic acid, oleic acid and stearic acid.

The term "gene transfer activity" as used herein refers to the activity of "gene transfer" by a vector, and may be detected using a function of the transferred gene as an index indicator (for example, expression of the encoded protein and/or activity of the protein, in the case of an expression vector).

The term "inactivation" as used herein refers to a virus with an inactivated genome. Inactivated viruses are replication defective. Preferably, the inactivation is achieved by UV treatment or treatment with an alkylation agent.

The term "foreign gene" as used herein refers to a nucleic acid contained in a viral envelope vector but not originating from the virus, or a nucleic acid contained in a liposome vector. In one aspect of the present invention, the foreign gene is operatively linked with a regulatory sequence which allows the gene transferred by a gene transfer vector to be expressed (e.g., a promoter, enhancer, terminator and a poly A addition signal are required for transcription, and a ribosome binding site, initiation codon and a termination codon are required for translation). In another aspect of the present invention, the foreign gene does not include a regulatory sequence for expression of the foreign gene. In a further aspect of the present invention, the foreign gene is an oligonucleotide or a decoy nucleic acid.

A foreign gene contained in a gene transfer vector is typically a nucleic acid of DNA or RNA, and the transferred nucleic acid molecule may include a nucleic acid analogue molecule. Molecular species contained in a gene transfer vector may be a single gene molecule species or a plurality of different gene molecule species.

The term "gene library" as used herein means a nucleic acid library including nucleic acid sequences isolated from the natural world or synthetic nucleic acid sequences. Examples of the source of nucleic acid sequences isolated from the natural world, include, but are not limited to, genome sequences and cDNA sequences derived from eukaryotic cells, prokaryotic cells, or viruses. A library of sequences isolated from the natural world to which optional sequences (e.g., signal sequences or tag sequences) are added is also encompassed in the gene library of the present invention. In one embodiment, a gene library also includes sequences such as promoter sequences that enable transcription and/or translation of the nucleic acid sequences contained in the library.

The terms "HVJ" and "Sendai Virus" as used herein are used interchangeably. For example, the terms "envelope of HVJ" and "envelope of Sendai Virus" are synonymously used herein. "Sendai Virus" as used herein belongs to the genus paramyxovirus in the family Paramyxoviridae, and has cell fusion activity. The viral particles are enveloped, and are polymorphic in that the particle diameter varies from 150 to 300 nm. The genome is a (-) strand RNAmolecule having a length of about 15500 bases. The virus has an RNA polymerase, is thermally unstable, hemagglutinates almost all types of erythrocyte and exhibits hemolytic activity.

The term "HAU" used herein refers to a measure of virus activity that is able to hemagglutinate 0.5% of chicken erythrocytes, wherein 1 HAU corresponds to about 24 million viral particles (Okada, Y. et al., Biken Journal 4, 209-213, 1961).

The term "candidate nucleic acid" as used herein may be any nucleic acid insofar as it is an obj ect to be purified. Herein, a population of candidate nucleic acids may be obtained directly from cells, a first host cell such as a mammalian cell as a source, or obtained as an isolated nucleic acid preparation. The population of candidate nucleic acids thus obtained is purified using a second host cell.

Examples of animal cells that may be used as a host cell include, but are not limited to, mouse myeloma cell lines, rat myeloma cell lines, mouse hybridoma cells, CHO cells which are derived from the Chinese hamster, BHK cells, African green monkey kidney cell lines, human leucocyte-derived cell lines, the cell line HBT5637 (Japanese Laid-Open Publication No. 63-299) and human colon cancer cell lines. Mouse myeloma cell lines include, ps20, NSO and the like. Rat myeloma cell lines include YB2/0 or the like. Human embryo kidney cell lines include HEK293 (ATCC: CRL-1573) or the like. Human leucocyte-dervied cell lines include BALL-1 or the like. African green monkey kidney cell lines include COS-1, COS-7 or the like. Human colon cancer cell lines include HCT-15 or the like.

The term "animal" as used herein is used in the broadest sense within the art and includes vertebrates and invertebrates. Examples of animals include, but are not limited to, the classes Mammalia, Aves, Reptilia, Amphibia, Pisces, Insecta, Vermes and the like.

The term "tissue" of an organism as used herein refers to a population of cells having a certain similar ability across the population. Therefore, the tissue may be a part of an organ. A particular organ often has cells having the same function, however, it may include cells having slightly different functions. Therefore, in this specification, a variety of cells may be included in a particular tissue insofar as they commonly have a certain characteristic.

The second host cell is not particularly limited insofar as it is a cell capable of "gene-transferring" a candidate nucleic acid; various host cells that are conventionally used in genetic engineering (for example, prokaryotic cells and eukaryotic cells) may be used.

Examples of prokaryotic cells include, prokaryotic cells belonging to the genus selected from the group consisting of Escherichia, Bacillus, Streptococcus, Staphylococcus, Haemophilus, Neisseria, Actinobacillus, Acinetobacter, Serratia, Brevibacterium, Corynetbacterium, Microbacterium, and Pseudomonas, for example, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 (DE3), Escherichia coli BL21 (DE3) pLysS, Escherichia coli HMS174 (DE3), Escherichia coli HMS174 (DE3) pLysS, Serratia ficaria, Serratia fonticola, Serratia liquefaciens, Serratia marcescens, Bacillus subtilis, Bacillus amyloliquefaciens, Brevibacterium ammmoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, Pseudomonas sp D-0110 and the like.

Examples of eukaryotic cells include, yeast strains belonging to the genus Saccharomyces, Schizosaccharomyces, Kluyveromyces, Trichosporon, Schwanniomyces, Pichia, and fungi belonging to Neurospora, specifically, Saccharomyces cerevisiae, Schizosaccharomyces pombe, Kluyveromyces lactis, Trichosporon pullulans, Schwanniomyces alluvius, Pichia pastoris and the like. As a method of transferring a recombinant vector in the host cells, any method for transferring DNA into fungi can be used, such methods include electroporation methods [Methods Enzymol., 194, 182 (1990)], spheroplast-based methods [Proc. Natl. Acad. Sci. USA, 84, 1929 (1978)], lithium acetate-based methods [J. Bacteriol., 153, 163 (1983)] and the method described in Proc. Natl. Acad. Sci. USA, 75, 1929 (1978).

Plant cells, for example, include plant cells derived from potato, tobacco, corn, rice plant, rapeseed, soy bean, tomato, carrot, wheat, barley, rye, alfalfa and flax. As a method of transferring a recombinant vector, any method for transferring DNA into a plant cell can be used. Examples of such methods include the use of Agrobacterium (Japanese Laid-Open Publication No. 59-140885, Japanese Laid-Open Publication No. 60-70080, WO94/00977), electroporation methods (Japanese Laid-Open PublicationNo. 60-251887) andmethodsusingaparticlegun (gene gun) (Japanese patent No. 2606856, Japanese patent No. 2517813) .

Insect cells may include Spodoptera frugiperda ovary cells, Trichopllusia ni ovary cells, cultured cells derived from silkworm ovaries and the like. Examples of Spodoptera frugiperda ovary cells include Sf 9, Sf21 (Baculovirus Expression Vectors: A Laboratory Manual) and the like, examples of Trichopllusia ni ovary cells include High 5, BTI-TN-5B 1-4 (Invitrogen) and the like, and examples of the culture cells derived from silkworm ovaries include Bombyx mori N4.

The term "variant" as used herein refers to substances which are partially modified from the original substances such as polypeptides or polynucleotides. A variant includes substitution variants, addition variants, deletion variants, truncated variants, allelic mutants and the like. Alleles are genetic variants which belong to the same locus but are distinct from each other. Therefore, the term "allelic gene mutant" means a variant which forms an allelic relative to a certain gene. The term "species homolog or homolog" denotes to that the substance has a homology (preferably, homology of 60% or more, more preferably, homology of 80% or more, 85% or more, 90% or more, 95% or more) with a certain gene in a certain species at the amino acid level or nucleotide level. A method for obtaining such a species homolog is apparent from the description of this specification. An "ortholog" is also called an "orthologous gene", the term is used for two genes that result from speciation of a specific common ancestor. Taking the hemoglobin gene family having a multigene structure as an example, human and mouse α-hemoglobin genes are orthologs, but the human α-hemoglobin and β-hemoglobin genes are paralogs (genes generated as a result of gene duplication). Since orthologs are useful for estimating a molecular phylogenetic tree, orthologs may also be useful in the present invention.

The term "conservative (conservatively modified) variant" is applicable both to amino acid sequences and nucleic acid sequences. Regarding a specific nucleic acid sequence, a conservative variant refers to a nucleic acid that encodes the same or substantially the same amino acid sequence. When the nucleic acid does not encode an amino acid sequence, it refers to substantially the same sequence. Because of the degeneracy of genetic codes, a large number of functionally equivalent nucleic acids encode any particular protein. For example, the codons GCA, GCC, GCG and GCU all encode the amino acid, alanine. Therefore, at every position where a codon specifies alanine, the codon may be changed into any corresponding codon described above without changing the encoded polypeptide. Such variation of nucleic acids is "silent variant (mutation)" which is one of the conservative variants. Any nucleic acid sequence encoding a polypeptide herein also describes all possible silent mutations for the nucleic acid. One skilled in the art will recognize that each codon in a nucleic acid (excluding AUG which is usually a unique codon for methionine, and TGG which is usually a unique codon for tryptophan) may be modified so as to produce a functionally identical molecule. Therefore, all possible silent mutations of a nucleic acid encoding a polypeptide is implied in each of the described sequences. Preferably, such modification may be made so as to avoid substitution of cysteine which is an amino acid that exerts great influence on the higher structure of polypeptides.

In order to create a functionally equivalent polypeptide, addition, deletion or modification of amino acid may be carried out herein besides substitution of amino acid. Substitution of an amino acid refers to making a substitution in the original peptide with at least one amino acid, for example 1 to 10, preferably 1 to 5, more preferably 1 to 3 amino acids. Addition of an amino acid refers to adding onto the original peptide, at least one amino acid, for example 1 to 10, preferably 1 to 5, more preferably 1 to 3 amino acids. Deletion of an amino acid refers to deleting from the original peptide, at least one amino acid, for example 1 to 10, preferably 1 to 5, more preferably 1 to 3 amino acids. Examples of amino acid modification include, but are not limited to, amidation, carboxylation, sulfation, halogenation, alkylation, glycosylation, phosphorylation, hydroxylation and acylation (for example, acetylation). Amino acids that are substituted or added may be naturally occurring amino acids, non-naturally occurring amino acids, or amino acid analogues. Naturally occurring amino acids are preferred.

Such a nucleic acid may be obtained using well-known PCR methods or chemical synthesis. Site-directed mutagenesis, hybridization methods and the like may be combined with the methods described above.

The term "substitution, addition or deletion" of a polypeptide or polynucleotide as used herein refers to the occurrence of substitution, addition or deletion of an amino acid or it's alternative, or a nucleotide or it's alternative from the original polypeptide or polynucleotide. Techniques for substitution, addition or deletion are well known in the art, and include, for example, site-directed mutagenesis and the like. The number of substitutions, additions or deletions is at least one, and any number is acceptable insofar as a function of interest (for example, a cancer marker, a neurological disease marker or the like) is retained in a variant having such a substitution, addition or deletion. For example, the number may be one or several, preferably within 20%, within 10% of the entire length, or less than or equal to 100, less than or equal to 50, or less than or equal to 25.

General molecular biological methods that may be used herein can be readily practiced by a person skilled in the art with reference to, for example, Ausubel F.A. et al. ed. (1988), Current Protocols in Molecular Biology, Wiley, New York, NY; SambrookJ. et al. (1987) Molecular Cloning: A Laboratory Manual, 2nd Ed. , Cold Spring Harbor Laboratory Press, Cold Spring Harbor, NY).

The term "expression plasmid" as used herein refers to a nucleic acid sequence in which, in addition to a structural gene and a promoter for regulating expression thereof, various regulatory elements are linked so as to be able to operate in a host cell. Preferably, the regulatory elements may include a promoter, a terminator and a selection marker. It is well-known by those skilled in the art that the type of expression plasmid and the kind of regulatory element used may vary depending on the host bacterial cell. In the present invention, an expression plasmid expresses a candidate nucleic acid in a first host cell and/or second host cell. Therefore, the expression plasmid includes a candidate nucleic acid, and a regulatory element (for example, promoter) operably linked with the candidate nucleic acid.

The term "promoter" used herein refers to a region of DNA that determines the transcription initiation site of a gene and directly regulates the frequency of transcription thereof, and a base sequence where RNA polymerase binds to start transcription. A putative promoter region varies with the particular structural gene, and is usually positioned upstream of a structural gene. Not limited to these, a putative promoter may also be positioned downstream of a structural gene.

A promoter may be inducible, constitutive, site specific, or period specific. As the promoter, any promoters that can be expressed in a host cell such as mammalian cells, Escherichia coli, yeast or the like are acceptable.

When used herein concerning the expression of a gene, the term "site specificity" generally refers to the specificity of expression of the gene within specific mammalian tissues. The term "period specificity" refers specifically to expression of the gene in accordance with the developmental stage of a mammal. Such specificity may be introduced into a desired organism by selecting an appropriate promoter.

When the expression of a promoter of the present invention is described as "constitutive" herein, it means that in all tissues in organism, the gene is expressed in an almost constant amount regardless of the growth/proliferation of the organism. More specifically, if during Northern blotting analysis almost the same level of expression is observed both in the same or in a corresponding site at given points (for example, two or more points (for example, at 5 days, and 15 days)) the expression is said to be constitutive according to the definition of the present invention. Constitutive promoters are believed to play a role in maintaining the homeostasis of organisms in normal growth environments. The term that the expression of a promoter of the present invention is "responsive" means that when at least one factor is given to an organism, the level of expression changes. In particular, when expression levels increase in response to at least one factor the expression is said to be "inducible" by the factor, and when expression levels reduce in response to at least one factor the expression is said to be "reductive" by the factor. "Reductive" expression is based on the premise the fact that expression is initially observed under normal conditions, and hence "reductive" expression is concept that overlaps "constitutive" expression. These properties may be determined by analyzing RNA extracted from a specific tissue of an organism in order to analyze the expression levels by Northern blotting analysis or by quantitating the expressed protein by Western blotting. A mammalian cell or a mammal (including a specific tissue) transformed with a vector that incorporates a promoter inducible by a factor, as well as a nucleic acid encoding a site specific recombinant inducing factor of the present invention, may be subjected to site specific recombination of the site specific recombination sequence under certain conditions by using a stimulating factor having the function of inducing the promoter.

As a potent promoter for expression in a mammalian cell, a variety of naturally occurring promoters (for example, the early promoter of SV40, the EIA promoter of adenovirus, the promoter of human cytomegarovirus (CMV), the human elongation factor-1 (EF-1) •promoter, the promoter of the Drosophila minimum heat shock protein 70 (HSP),the human metallothionein (MT) promoter, the Rous-sarcoma virus (RSV) promoter, the human ubiquitinC (UBC) promoter, human act in promoter), and artificial promoters (for example, fusion promoters such as SRα promoter (a fusion of the SV40 early promoter and the LTR promoter of HTLV) and the CAG promoter (a hybrid of the CMV-IE enhancer and the chicken act in promoter)) are well known. Therefore, by using these well-known promoters or variants thereof, it is possible to readily increase the expression level .

When Escherichia coli is used as a host cell, promoters derived from Escherichia coli or phages such as the trp promoter (Ptrp), the lac promoter (Plac), the PL promoter, the PR promoter, the PSE promoter, the SPO1 promoter, the SPO2 promoter, the penP promoter and the like can be exemplified. Also artificially designed and modified promoters such as a promoter comprising two serially linked Ptrps (Ptrp x2), the tac promoter, the lacT7 promoter, the let I promoter and the like may be used.

The term "enhancer" may be used herein for improving the expression efficiency of a gene of interest. A typical enhancer when used in a mammalian cell includes, but is not limited to, an enhancer of SV40. An enhancer may be used singly or in plural, or may not be used at all.

The term "terminator" as used herein refers to a sequence that is positioned downstream of a protein coding region of a gene, and is involved in the termination of transcription and the addition of a poly A tail when DNA is transcribed into mRNA. A terminator is known to be involved in the stability of mRNA and to influence the expression level of a gene.

The term "operably linked" as used herein means that expression (operation) of an intended sequence is placed under the control of a transcription and translation regulatory sequence (for example, a promoter, an enhancer or the like) or under a translation regulatory sequence. In order to operably link a promoter to a gene, usually, the promoter is located directly upstream of the gene, however, it is not necessarily located adjacently.

As used herein, the term "biological activity" is used interchangeably with the term "functional property". The terms "biological activity" and "functional property" as used herein mean an activity that a certain factor (for example, a nucleic acid) may have in an organism, and encompasses activities exerting various functions. For example, when a certain factor is a gene encoding a growth factor, the functional property encompasses expressing the growth factor in a host cell, and preferably promoting the growth of the cell by expression of the growth factor. For example, when the certain factor is a gene encoding an enzyme, the functional property encompasses expressing the enzymatic activity in a host cell and preferably increasing enzymatic activity to a detectable level. In another example, when the certain factor is a gene encoding a ligand, the functional property encompasses expressing a ligand that binds a receptor corresponding to the ligand, and preferably changes the phenotype of a cell having the receptor corresponding to the ligand, by the expression of the ligand.

In this specification, when it is necessary to transfer a nucleic acid into a second host cell, anymethod for transferring DNA into a host cell canbe used. Examples of such methods include, transfection, transduction, transformation (for example, electroporation , methods using a particle gun (gene gun) and the like).

When referring to gene herein, the term "expression plasmid" means a nucleic acid capable of expressing a gene included in a polynucleotide sequence of interest after the polynucleotide sequence of interest is transferred into a cell of interest. Expression plasmids include plasmids having a promoter at a position suited for transcription of the polynucleotide to be expressed.

In this specification, "detection" or "quantification" of the expression of a nucleic acid transferred into a host cell may be achieved by using appropriate methods including measurement of mRNA and immunological measuring methods. Examples of molecular biological measuring methods include Northern blotting, Dot blotting, PCR and the like. Examples of immunological measuring methods include ELISA using a micro titerplate, RIA, fluorescent antibodymethods, Westernblotting, immunohistochemical staining and the like. Quantification methods include ELISA or RIA.

In this specification, "detection" or "quantification" of expression of nucleic acids transferred into a host cell may be carried out using a solid phase (for example, a substrate, support, array, chip or microchip).

The terms "substrate" and "support" are used in the same meaning herein, and refer to a material (preferably solid) from which an array of the present invention is constructed. A material for the substrate includes any solid material having the characteristic of binding a biological molecule used in the present invention via a covalent or noncovalent bond or any material that can be derivatized to have such a characteristic.

Materials used for a substrate include any material capable of forming a solid surface, and include, but are not limited to, for example, glass, silica, silicones, ceramics, silicon dioxide, plastics, metals (including alloy), naturally occurring or synthetic polymers (for example, polystyrene, cellulose, chitosan, dextran and nylon). A substrate may have a plurality of layers formed of different materials. For example, inorganic insulating materials, such as glass, quartz glass, alumina, sapphire, forsterite, silicon carbide, silicon oxide, silicon nitride and the like may be used. Also organic materials such as polyethylene, ethylene, polypropyrene, polyisobutylene, polyethylene terephtalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, silicone resin, polyphenylene oxide, polysulfone and the like may be used. In the present invention, membranes that are used for blotting such as nylon membrane, nitrocellulose membrane, PVDF membrane and the like may be used. A nylon membrane is preferred because the result may be analyzed using a convenient analyzing system when a nylon membrane is used. However, when an object of high density is analyzed, the use of hard materials such as glass is preferred.

The term "chip" or "microchip" as used herein refers to an ultrasmall integrated circuit which has a plurality of functions and forms a part of a system. As used herein, a "DNA chip" includes a substrate and DNA, and at least one DNA molecule (for example, cDNA fragment) is placed on the substrate. As used herein, a "protein chip" includes a substrate and protein, and at least one protein (for example, a polypeptide or oligopeptide) is placed on the substrate. As used herein, a "DNA chip" and a "protein chip" are encompassed by the terms "microchip" or simply "chip". The term "microarray" means a chip on which at least one biological molecule (for example, an oligonucleotide such as a cDNA fragment, or a peptide) is placed in array.

A biological molecule (for example, an oligonucleotide such as a cDNA fragment or a peptide) as used herein may be collected from an organism ormaybe chemically synthesized using methods known by those skilled in the art. For example, oligonucleotides may be prepared by automated chemical synthesis using either a DNA synthesizer or a peptide synthesizer commercially available from Applied Biosystems or the like. Compositions and methods for automated oligonucleotide synthesis are disclosed in, for example, USP No. 4,415,732, Caruthers et al. (1983) ; USP No. 4, 500, 707 and Caruthers (1985); USP No. 4,668,777, Caruthers et al. (1987).

On a substrate, any number of biological molecules (for example, DNA molecules or peptides) may be placed, and usually up to 10⁸ biological molecules, and in another embodiment, up to 10⁷ biological molecules, up to 10⁶ biological molecules, up to 10⁵ biological molecules, up to 10⁴ biological molecules, up to 10³ biological molecules, or up to 10² biological molecules may be placed on one substrate. In these cases, the size of the substrate is preferably as small as possible. In particular, the size of a spot of a biological molecule (for example, a DNA molecule or a peptide) may be as small as the size of a single biological molecule (i.e., in the order of 1-2 nm). In some cases, the minimum substrate area is determined by the number of biological molecules on the substrate.

The term "biological molecule" as used herein refers to molecules associated with organisms. As used herein, the term "organisms" refers to a biological organism including, but not limited to, animals, plants, fungi and viruses. The term "biological molecules" encompasses molecules extracted from organisms, however, is not limited to this, and any molecule that influences an organism is encompassed by the definition of a biological molecule. Examples of such biological molecules include, but are not limited to, proteins, polypeptides, oligopeptides, peptides, polynucleotides, oligonucleotides, nucleotides, nucleic acids (including, for example, DNA molecules such as cDNA and genomic DNA, and RNA molecules such as mRNA, polysaccharides, oligosaccharides, lipids, small molecules (for example, hormones, ligands, signal transducers, organic small molecules and the like), and composite molecules thereof. Asused herein,biologicalmoleculesmay be preferably peptides, DNA or RNA.

In the case where a host cell changes due to a nucleic acid transferred into the host cell, it is possible to "detect" or "quantify" the expression of the nucleic acid transferred into the host cell by measuring the degree of the change. Examples of such changes in host cells include, but are not limited to, changes in enzymatic activity of a specific enzyme in a cell, changes in cell growth rate, and the like.

The term "expression amount" refers to the amount in which a polypeptide or mRNA is expressed in a cell of interest. Such an expression amount may be the level of protein expression of the polypeptide of the present invention evaluated by any appropriate method, including immunological measuring methods such as ELISA, RIA, fluorescent antibodies, Western blotting and immunohistochemical staining using a antibody of the present invention; or the level of mRNA expression of the polypeptide of the present invention evaluated by any appropriate methods, including molecular biological methods such as Northern blotting, dot blotting, and PCR and the like. As used herein, "expression amount" may be an absolute value represented by a numerical unit, such as expressed weight, absorbance having correlation with expressed weight and the like, or may be a relative value represented by a ratio relative to a control or comparison reference. "Change in expression amount" means increase or decrease in the expression amount in the protein level or in the mRNA level of the polypeptide of the present invention as evaluated by any appropriate method including the forgoing immunological or molecular biological methods.

### DESCRIPTION OF PREFERRED EMBODIMENTS

In the present invention, a method of isolating a nucleic acid having an intended functional property from candidate nucleic acids can be carried out by the following steps:
(A) transferring a nucleic acid into a plurality of first host cells and allowing the nucleic acid to transiently express therein;
(B) selecting, from the plurality of first host cells into which the nucleic acid is transferred, a cell having the nucleic acid of the intended functional property transferred therein;
(C) preparing a purified nucleic acid from the selected cell; and
(D) selecting a purified nucleic acid having the intended functional property.

In the above method, the candidate nucleic acids may be derived from any organism, and may be DNA, RNA or nucleotide analogues. The candidate nucleic acids may be one kind or a plurality of kinds.

In the above methods, the first host cells are preferably animal cells, more preferably mammalian cells including human cells, although not particularly limited thereto. The candidate nucleic acids may be transferred into the first host cells using a variety of known methods. Typical methods include, but are not limited to, a transferring method using a viral envelope, a transferring method using a liposome, a transferring method using a liposome containing at least one protein from a viral envelope, a transferring method using calcium phosphate, and an electroporation method. When the transferring method using a viral envelope or the transferringmethodusing a liposome containing at least one protein from a viral envelope is used, the virus used for preparing the viral envelope may be derived from viruses belonging to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae. Preferably, the virus is viruses of the family Paramyxoviridae, particularly HVJ.

A method of transferring candidate nucleic acids into first host cells using a liposome containing at least one protein from viral envelope is also available. Examples of the protein used in this method include, but are not limited to, F protein, HN protein, NP protein, M protein, or a combination thereof.

The methods for transferring the candidate nucleic acids into first host cells are also applicable to second host nucleic acids and third host nucleic acids.

Moreover, after transferring candidate nucleic acids into first host cells, mutation may be induced in the first host cells to increase the diversity of the nucleic acids transferred in the host cells. Mutagenesis methods for cells are well known, and examples of such methods include, but are not limited to, methods using chemicals such as ethidium bromide and nitrosoguanidine, and physical methods such as UV irradiation, X-ray irradiation and radioactive ray radiation. In the present invention, after increasing the diversity through mutagenesis, a candidate nucleic acid having the intended functional property may be selected from the resultant mutants.

When candidate nucleic acids are expressed in the first host cells, the candidate nucleic acids may be operably linked with a regulatory element such as promoter. This expression may be transient or stable.

Transient expression of candidate nucleic acids in the first host cells is sufficient. However, the host cells may allow stable expression of the candidate nucleic acids after the transient expression of candidate nucleic acids occurs. Such a host cell is also within the scope of the present invention.

Next, from the host cells into which the nucleic acids are transferred, a cell into which a nucleic acid having the intended functional property has been transferred is selected. The intended functional property is selected, for example, from the group consisting of induction of angiogenesis, tumor suppression, enhancement of osteogenesis, induction of apoptosis, cytokine secretion, induction of dendrites, suppression of arteriosclerosis, suppression of diabetes; suppression of autoimmune diseases; suppression of Alzheimer's disease, suppression of Parkinson's disease, protection of nerve cells and combinations thereof

Preferably, this selection is effected based on the phenotype of a host cell which changes in response to expression of candidate nucleic acids. For example, when a gene encoding a growth factor is isolated from candidate nucleic acids, the intended functional property is promotion of growth of a specific cell or any cells.

The method of the present invention may be applicable to any functional properties as long as the functional property of interest is recognizable. Examples of functional properties intended in the present invention include, but are not limited to, promotion or suppression of cell growth; differentiation or de-differentiation of cells; expression of marker proteins or suppression of the expression of marker proteins; expression of marker mRNA or suppression of expression of marker mRNA; change in membrane potential; depolarization; apoptosis; carcinogenesis; arrest of growth; change in morphology; change in size and the like.

Regarding the method for preparing a purified nucleic acid from a cell that is selected as containing a nucleic acid having the intended functional property, the second host cell may or may not be used. When the second host cell is used, the method is carried out in the following steps without limitation:
(i) extracting a nucleic acid from the selected cell;
(ii) transferring the extracted nucleic acid into a second host cell to thereby obtain a transformed cell;
(iii) purifying the transformed cell; and
(iv) preparing a nucleic acid from the purified, transformed cell.

In the foregoing method, a variety of well-known methods and commercially available kits may be used to extract nucleic acids from the first host cells. Depending on the kind of the second host cell, various well-known methods may be applied so as to transfer the nucleic acid to the second host cell. For example, when a bacterium is used as the second host cell, gene transfer may be achieved in the following manner without any limitation: preparing competent cells by a calcium method or the like, and transferring nucleic acids into bacterial host cells by the application of heat shock. Electroporation may also be used. Non-limiting examples of methods for transferring DNA into fungi include the use of electroporation [Methods. Enzymol., 194, 182 (1990)], spheroplasts [Proc. Natl. Acad. Sci. USA, 84, 1929 (1978)], and lithium acetate. When a plant cell is used as a host cell, known methods include, but are not limited to, the use of Agrobacterium (Japanese Laid-Open Publication No. 59-140885, Japanese Laid-Open Publication No. 60-70080, WO94/00977), electroporation methods (Japanese Laid-Open PublicationNo. 60-251887) and methods using a particle gun (gene gun). When an animal cell is used as a host cell, available methods include but are not limited to, a transferring method using a viral envelope, a transferring method using a liposome, a transferring method using a liposome containing at least one protein from viral envelope, a transferring method using calcium phosphate, and electroporation methods.

In the case where a second host cell is used, preferably only one kind of candidate nucleic acid per cell is transferred into the second host cell. Therefore, when a population of candidate nucleic acids is obtained, the population is transferred into the second host cell, and a host cell having a nucleic acid transferred therein is purified, whereby the candidate nucleic acid can be purified.

If a plurality of kinds of purified nucleic acids are observed after purification of the nucleic acids, a purified nucleic acid having the intended functional property may be selected from the purified nucleic acids. This selection may be achieved by expressing the purified nucleic acid, confirming the function of the nucleic acid, and determining whether or not the confirmed function is the intended function. Alternatively, it may be achieved by sequencing the whole or a part of the purified nucleic acid structure.

Kits for practicing the methods of the present invention are also provided in the present invention.

### (1. Preparation of a viral envelope vector)

Various methods for preparing a viral envelope vector are known in the art. For example, the present inventors developed a hybrid gene transfer vector by combining a viral vector and a non-viral vector, and constructed a fusion forming viral liposome having a fusion forming envelope derived from hemagglutinating virus of Japan (HVJ: Sendai Virus) (Kaneda, BiogenicAmines, 14:553-572 (1998) ; Kaneda et al, Mol. Med. Today, 5:298-303 (1999)). In this delivery system, a liposome filled with DNA is fused with UV inactivated HVJ, to thereby form a HVJ liposome which is a fusion forming viral liposome (diameter: 400-500 nm). Fusion-mediated delivery is advantageous in that transfection of DNA is protected from endosomal degradation and lysosomal degradation in the recipient cell. DNA having a length of up to 100 kb is incorporated into an HVJ liposome, and delivered into a mammalian cell. RNA, oligonucleotides and drugs are also introduced into a cell efficiently in vitro or in vivo. HVJ-liposome was not shown to induce significant cell damage in vivo.

Repeated transfection in vivo has succeeded due to the low immunogenicity of HVJ (Hirano et al., Gene Ther. , 5: 459-464 (1998)). This vector system was modified, and anion type and cation type HVJ-liposomes have been developed for more efficient gene delivery (Saeki at al., Hum. Gene Ther., 8:1965-1972 (1997)). Using this HVJ-liposome system, a great number of gene therapy strategies have succeeded (Dzau et al, Proc. Natl. Acad. Sci. USA, 93:11421-11425(1996); Kaneda et al., Mol. Med. Today, 5:298-303 (1999)). Several attempts to construct HVJ-derived synthetic virosomes have also been made (Wu et al., Neuroscience Lett., 190:73-76 (1995)); Ramanietal., FEBSLett., 404:164-168 (1997) ; Ramani et al. , Proc. Natl. Acad. Sci. USA, 95:11886-11890 (1998)).

When it is necessary to inactivate a virus for preparing a vector, a variety of known methods may be used. Typical inactivation methods include, but are not limited to, UV irradiation, treatment with an alkylation agent, treatment with β-propiolacton, treatment with surfactant, and partial degradation of the envelope by enzymatic treatment.

Modifications to the foregoing methods of preparing a viral envelope vector are also known. Typical examples are shown below. The method of preparing a viral envelope vector shown below is only for illustration, and the present invention is not limited to vectors that are prepared in the method described below.

### (1.1. Preparation of a gene transfer vector encapsulating a foreign gene in a component containing a protein derived from viral envelope)

Examples of a gene transfer vector containing a protein derived from a viral envelope include, but are not limited to, gene transfer vectors consisting of liposomes obtained by reconstitution of the F fusion protein and HN fusion protein of HVJ (Sendai Virus), but not including an amount of HVJ genomic RNA that is detectable by RT-PCR.

The F fusion protein and HN fusion protein used in preparation of such a gene transfer vector may be protein of naturally-occurring HVJ or recombinantly expressed protein. Recombinantly produced fusion proteins are subjectedto in vitro processing with proteases, or processing with endogenous proteases in a mammalian host cell.

A gene transfer vector containing a protein derived from a viral envelope is prepared, for example, by a method comprising the following steps:
- isolating a fusion protein from HVJ virus that has not been irradiated with UV;
- reconstituting the fusion protein in the presence of a surfactant and a lipid to prepare a reconstituted particle;
- preparing a liposome filled with the intended nucleic acid; and
- fusing the reconstituted particle and the liposome.

The surfactant used in the above method is not particularly limited to a specific surfactant, and preferred examples include octylglucoside, Triton-X100, CHAPS or NP-40, or mixtures thereof.

The lipid used in the above method is not particularly limited to a specific lipid, and may be those (1) having a long chain fatty acid or a similar hydrocarbon chain in the molecule, and (2) naturally occurring in an organism or derived from an organism. Preferred examples of the lipid include, but are not limited to, phosphatidyl choline, phosphatidyl serine, cholesterol, sphingomyelin, and phosphatidic acid.

A preparation method of liposomes is well known, and for example, the following method may be used:
(A) Prepare a thin film of phospholipids in a first test tube in advance. Nitrogen gas saturated with water at 55°C is introduced to this test tube and allowed to sufficiently hydrate the thin film of phospholipids. Upon completion of hydration, the thin film turns transparent.
(B) To the test tube A, a buffer of from a second test tube is added smoothly, and after introduction of nitrogen gas, the test tube is sealed (for example, with Parafilm) and kept in an incubator (which is an apparatus that enables the experiment to be conducted at a constant temperature) at 37°C for about two hours.
(C) Macro-liposomes are prepared by gentle shaking. As a result of generation of liposomes, the liquid becomes slightly cloudy. Using this, the following measurement is conducted.
(D) Placing a droplet of sample on a slide glass, the morphology of the liposomes is observed under a fluorescence microscope (x1000).

A method of preparing a protein that is required for preparing a gene transfer vector encapsulating a foreign gene in a component containing a protein derived from a viral envelope is well known.

For example, it may be prepared by purification of HVJ envelope protein from naturally occurring HVJ, or purification of recombinantly expressed HVJ envelope protein. Examples of well-known protein purification methods include, but are not limited to, ammonium sulfate precipitation, electrofocusing, and purification using a column. When a protein is purified using a column, various columns may be selected depending on the properties of the intended protein and the properties of likely contaminants. Examples of columns used for protein purification include, but are not limited to, an anion exchange column, a cation exchange column, a gel filtration column and an affinity column.

Alternatively, a gene transfer vector containing a protein derived from a viral envelope is prepared by a method comprising the steps of:
- recombinantly expressing the F protein and HN protein of HVJ;
- processing F protein with a protease;
- isolating F protein and HN protein;
- reconstituting F protein and HN protein in the presence of a surfactant and lipids to prepare a reconstituted particle;
- preparing a liposome filled with nucleic acid; and
- fusing the reconstituted particle and the liposome.

A gene transfer vector containing a protein derived from a viral envelope may also be prepared by a method comprising the steps of:
- recombinantly expressing F protein and HN protein, in a host cell in which a protease that processes F protein is expressed;
- isolating F protein and HN protein;
- reconstituting F protein and HN protein in the presence of a surfactant and lipids to prepare a reconstituted particle;
- preparing a liposome filled with an intended nucleic acid; and
- fusing the reconstituted particle and the liposome.

### (1.2. Preparation of a gene transfer vector encapsulating a foreign gene in a viral envelope)

One exemplary method of preparing a gene transfer vector encapsulating a foreign gene in a viral envelope comprises the following steps:
1) mixing a virus and a foreign gene; and
2) freezing and thawing the mixture, or mixing the mixture with a surfactant.

Alternatively, a gene transfer vector derived from viral envelope can be prepared by a method comprising the steps of:
- inactivating a virus;
- mixing the inactivated virus with a foreign gene; and
- freezing and thawing the mixture.

In a further aspect of the present invention, there is provided a method of preparing an inactivated virus envelope vector for gene transfer, comprising the steps of:
- inactivating a virus; and
- mixing the inactivated virus with a foreign gene in the presence of a surfactant.

### (1.3. Liposome vector)

A liposome vector may use liposomes prepared from lipids commonly used in lipofection. For example, lipids such as lipofect AMINE 2000 may be used.

### (Selection of cells)

In the present invention, a variety of cells may be used as the first host cell. The first host cells are preferably mammalian cells and more preferably cells derived from the species from which the candidate nucleic acid is derived.

In the present invention, a variety of cells may be used as the second host cell. Any kind of cells may be used as the second host cell. Cells suited for the second host cell will incorporate one kind of candidate nucleic acid per cell. Examples of such cells include, but are not limited to, bacterial and fungal cells.

In the present invention, a variety of cells may be used as the third host cell. The third host cell is preferably the same as the first host cell, or a cell having a similar gene expression mechanism.

The present invention has now been illustrated with its preferred embodiments. The present invention will further be illustrated based on the Examples and referring to the drawings attached hereto. It should be noted that the following Examples are provided by way of illustration, and are not intended to limit the present invention. Therefore, the scope of the present invention is not limited to any specific embodiments recited by the examples below, and is only defined by the attached claims.

### EXAMPLES

### (Example 1: Preparation and use of a gene transfer vector encapsulating a foreign gene and a component containing a protein derived from viral envelope)

### (Preparation of virus)

HVJ, Z strain, was purified by differential centrifugation as previously described (Kaneda, Cell Biology: A Laboratory Handbook, J. E. Cells (Ed.), Academic Press, Orlando, Florida, vol. 3, pp. 50-57 (1994)). The purified HVJ was resuspended in a buffered salt solution (BSS: 137 mM NaCl, 5.4 mM KCl, 10 mM Tris-HCl, pH7.5), and the virus titer was determined by measuring absorbance at 540 nm. Optical density at 540 nm corresponds to 15,000 haemocyte aggregation unit (HAU) and correlates with fusion activity.

### (Extraction of F and HN fusion protein from HVJ)

Nonidet P-40 (NP-40) and phenylmethylsulfonyl fluoride (PMSF) dissolved in ethanol were added to 20 mL of suspension of purified HVJ (1, 750, 000 HAU), at final concentrations of 0.5% and 2 mM, respectively. The mixture was incubated at 4°C for 30 minutes with mixing. Then, the suspension was centrifuged at 100,000g, 4°C for 75 minutes to remove insoluble proteins and virus genome (Uchida et al., 1979). The supernatant was dialyzed against 5 mM phosphate buffer (pH 6.0) for three days, and the remaining NP-40 and PMSF were removed off by exchanging the buffer every day. The dialyzed solution was centrifuged at 100,000g, 4°C for 75 minutes, thereby removing insoluble substances. The supernatant was applied to an ion exchange column of CM-Sepharose CL6B (Pharmacia Fine Chemicals, Uppsala, Sweden) that was equilibrated with 10 mM phosphate buffer (pH 5.2) containing 0.3 M sucrose and 1 mM KCl, according to the previously described method (Yoshima et al., J. Biol. Chem., 256:5355-5361 (1981)). Flow-through fractions and the 0.2 M NaCl eluate were collected. These fractions were subjected to sodium dodecyl sulfate polyacrylamide gel electrophoresis (SDS-PAGE) and analyzed for protein components. The gel was stained with Coomassie brilliant blue, and the ratio of each protein was evaluated using computerized densitometry (NIH Image; Apple Computers, Cupertino, CA, USA).

### (Recombinant expression)

A fusion protein of HVJ may be prepared by incorporating a gene coding the fusion protein into an expression vector and expressing the gene in an appropriate host cell. The amino acid sequences of F protein and HN protein are known.

Expression vectors that may be used in various host cells are commercially available.

An expression vector encoding a fusion protein that may be transferred into a cell and subsequent production of a fusion protein of the present invention is known in the art, and can be carried out by any of the various described methods (for example, Sambrook et al., Molecular Cloning: A Laboratory Manual, 2nd Ed, Vols. 1 to 3, Cold Spring Harbor Laboratory Press, New York (1989) and Ausubel et al., Current Protocols in Molecular Biology, John Wiley and Sons, Baltimore, MD (1994), each incorporated herein by reference)). Examples of methods for transferring a recombinant expression vector into a prokaryotic or a eukaryotic cell include electroporation methods, transformation methods, or transfection methods.

When the recombinant Fprotein was expressed in Escherichia coli, it was expressed in an inactive F0 form. In order to convert the inactive F0 form of protein expressed in Escherichia coli into an active F1 form, trypsin treatment at 37°C for 30 minutes using 0.0004-0.001% trypsin was required.

The polypeptide corresponding to activated F1 protein treated with trypsin may be expressed in Escherichia coli using an expression vector containing a gene encoding the amino acid sequence of a truncated, activated F1. The truncated F1 protein necessarily includes at least 26 amino acids from phenylalanine, at position 117, to alanine, at position 142. In the case where the truncated protein forms an inclusion body, those skilled in the art can readily obtain an active form of the protein by refolding the inclusion body (see Robert F. Kelley and Marjorie E. Winkler, Genetic Engineering, (1990) vol. 12, pp.1-19 for reference).

When F protein is expressed by using cells in which HVJ can replicate (for example, rodent tracheal epithelium; chicken embryos; f monkey kidney primary culture cells; human fetal lung primary culture cells, kidney and amnion) as a host cell, the expressed full length F protein is cleaved by an endogenous protease of the host cell, and as a result, is activated. In this manner, an active form of F protein can be expressed and isolated. Alternatively, host cells in which Tryptase clara (Kido et al., Molecular Cells 9, 235-244 (1999)) is expressed as an endogenous enzyme (for example, rat tracheal epithelium) or host cells in which Tryptase clara is recombinantly expressed may also be used.

Methods of selecting and constructing these expression vectors, methods of transferring into host cells, methods of expressing in host cells, and methods of collecting expressed proteins are well known by those skilled in the art.

### (Purification of fusion protein from HVJ)

In order to purify a fusion protein, a lysate of HVJ treated with NP-40 was clarified by ultracentrifugation. Proteins in the supernatant were analyzed by SDS-PAGE prior to further purification. This supernatant containedmanyproteins derived from HVJ. Then, the supernatant was applied to an ion exchange column. Proteins of 52 kDa and 72 kDa were dominantly eluted in the flow-through fraction. These two proteins were identified as F1 and HN, respectively, based on their mobility in an SDS-PAGE gel (Okada, Methods in Enzymology, N. Duzgnes (Ed.), Academic Press; San Diego, vol.221, pp.18-41 (1993)). A minor band under the 52 kDa protein was considered to be a degradation product of the fusion proteins (Fl and HN). This is because these proteins were not reproducibly observed in different experiments. Proteins were further eluted with 0.2 M NaCl. However, fusion proteins were not obtained efficiently. Furthermore, a protein of 60 kDa that is speculated to be NP protein of HVJ additionally appeared. Eventually, only the flow-through fraction was used as a source of fusion proteins for subsequent experiments. Densitometry indicated that the F1 to HN concentration ratio in the flow-through fraction was 2.3:1. This is consistent with the ratio of these proteins in the viral envelope. A previous paper (Nakanishi et al., Exp. Cell Res., 142:95-101 (1982)) reported that this ratio is required for efficient fusion of HVJ.

### (Preparation of a gene transfer vector)

A lipid mixture consisting of 3.56 mg of phosphatidyl choline and 0.44 mg of cholesterol was dissolved in chloroform, and the resultant lipid solution was evaporated in a rotary evaporator (Uchida et al. , J. Cell. Biol. 80:10-20 (1979)). The dry lipid mixture was completely dissolved in 2.0 mL of protein solution (1.6mg) from the above flow-through fraction containing 0.85% NP-40 using a Vortex mixer.

This solution was then dialyzed against 10 mM phosphate buffer (pH 7.2) containing 0.3 M sucrose and 1 mM KCl to thereby remove NP40. The dialysis was continued for 6 days, and the buffer was replaced every day. The dialyzed solution was applied to agarose beads (Bio-Gel A-50m) (Bio-Rad Laboratories, Hercules, CA, USA) and equilibrated with 10 mM phosphate buffer (pH5.2) containing 0.3 M sucrose and 1 mM KCl. Fractions having an optical density of more than 1.5 at 540 nm were collected as reconstituted fusion particles. The gene transfer vector was prepared by fusing the reconstituted fusion particles with a liposome filled with nucleic acid prepared from 1. 0 mg of lipids as described below.

### (Expression of luciferase gene in HEK293 strain-derived transfected cells)

In order to confirm the gene transferring activity of the gene transfer vector prepared in the aforementioned manner, HEK293 cells and a luciferase gene were used in the following manner.

pCMV-luciferase (7.4 kb) was constructed by cloning a luciferase gene from pGEM-luc (Promega Corp., Madison, WI, USA) into pcDNA3 (5.4 kb) (Invitrogen, San Diego, CA, USA) at Hind III and Bam HI sites. A gene transfer vector containing about 40 µg of pCMV-luciferase was constructed in the manner described above, and 1/10 amount (100 µL) of the gene transfer vector (about 1.5x10¹¹ particles/mL, DNA concentration about 40 µg/mL) was incubated with 2x10⁵ cells derived fromhuman 293 cell line (human embryonic kidney: HEK). Using HVJ liposomes, the same amount of luciferase DNA was transferred into 2x10⁵ HEK293 cells. Twenty four hours after transduction, the cells were collected, and assayed for luciferase activity in the described manner (Saeki et al., Hum. Gene Ther., 8: 1965-1972 (1997)).

### (Example 2: Preparation of HVJ envelope vector by freezing and thawing and uses thereof)

### (Preparation and use of a gene transfer vector)

### (1: Preparation of HVJ envelope vector by freezing and thawing)

A recombinant HVJ virus containing a luciferase gene as a foreign gene, was subjected to various cycles of freezing and thawing before being transferred into a cultured cell.

Five hundred µL of TE, 750 µg of luciferase expression vector pcOriPLuc (Saeki and Kaneda et al., Human Gene Therapy, 11, 471-479 (2000)) and various concentrations of HVJ virus were mixed. HVJ virus was prepared in concentrations of 10, 25, 50 and 100 HAU/µL. The resultant solution was divided into 12 aliquots, and each aliquot was subjected to up to 30 cycles of freezing and thawing, each cycle consisting of freezing by storing at 4°C, frozen with dry ice and thereafter thawed; this was repeated up to 30 times. The resultant solution, having been subjected to a predetermined number of freezing and thawing cycles, was added to the medium of BHK-21 cells (4×10⁴ cells/dish, 0.5 mL DMEM, 10%FCS per 24-well dish), allowed to react at 37°C under 5% CO₂ for 20 minutes, the cells were washed with PBS, and then 0.5 mL of culture medium was freshly added and the cells cultured for 24 hours.

After removing the medium, 500 µL of 1×Cell Culture Lysis Reagent (Promega) was added to the cells to lyse the cells, and the resulting cell suspension was centrifuged in a micro tube. Twenty µL of the obtained supernatant was measured for luciferase activity using the Promega Luciferase Assay System and Lumat LB9501 Luminophotometer. The measurement was conducted three times for each solution, and an average value was determined.

As a result, it was observed that luciferase activity increased with the number of cycles of freezing and thawing of the recombinant HVJ virus. Upon 20 cycles of freezing and thawing, ten fold or more luciferase expression was observed as compared to 3 cycles of freezing and thawing. This result revealed that the number of cycles of freezing and thawing of recombinant HVJ virus is preferably 5 or more, more preferably about 15 to 20 under the conditions of the present Example.

### (2: Gene transferring efficiency of HVJ envelope vector prepared by freezing and thawing)

After 30 cycles of freezing and thawing of the recombinant HVJ virus, similar to that shown in Example 1 above, the transfer efficiency of a gene into cells was examined under the condition wherein the number of viruses added to the host cells was constant.

For example, in the case where the X axis is 500 HAU, 50 µL of a solution having a virus concentration of 10-50 HAU/µL, and 5 µL of a solution having a virus concentration of 100 HAU/µL were used. The efficiency of gene expression for a solution having a virus concentration of 100 HAU/µL was lower by about 50% compared with that for a solution having a virus concentration of 10-50 HAU/µL. This result revealed that under the conditions of this example, the recombinant virus concentration was preferably in the range of 10 to 50 HAU/µL.

After 29 cycles of freezing and thawing of the recombinant HVJ virus, the 30th freezing was conducted, the recombinant virus stored frozen for one week and then thawed before being added to cells. The recombinant HVJ virus stored frozen for one week and the recombinant HVJ virus subj ected to a continuous 30 cycles of freezing and thawing showed similar levels of luciferase gene expression.

### (Example 3: Preparation of an inactivated HVJ envelope vector utilizing a detergent)

### (1: Growth of HVJ)

In general, HVJ cultured by inoculating a fertilized chicken egg with seed virus may be used. However, HVJ grown in cultured cells (e.g., simian or human) or a persistent infection system (i.e., a culture medium supplemented with a hydrolase such as trypsin is added to cultured tissue), or HVJ grown by infecting cultured cells with cloned virus genome to cause persistent infection are applicable.

In the present example, the growth of HVJ was performed as follows.

HVJ seed virus was cultured in a SPF (Specific Pathogen Free) fertilized egg. The isolated and purified HVJ (Z species) was dispensed into a cryo-vial, DMSO added to 10%, and stored in liquid nitrogen.

Chicken eggs were obtained immediately after fertilization, and placed in an incubator (SHOWA-FURANKI P-03 type; capable of accommodating about 300 chicken eggs), and incubated for 10 to 14 days at 36.5°C and 40% or more humidity. In a darkroom, the viability of the embryo as well as the air cell and the chorioallantoic membrane was confirmed using an egg tester (specifically, an egg-tester in which light from a light bulb is projected through a window having a diameter of about 1.5 cm). A virus-inj ection site was marked in pencil about 5 mm above the chorioallantoic membrane (the position was selected so as to avoid any thick blood vessels). The seed virus (which was removed from liquid nitrogen) was diluted 500-fold with a polypeptone solution (1% polypeptone, 0.2% NaCl, adjusted to pH 7.2 with 1 M NaOH, then autoclave-sterilized and stored at 4°C), and left at 4°C. The egg was disinfected with Isodine™ and alcohol. A small hole was made in the virus-injected site with a pick. Using a 1 ml syringe and a 26 gauge needle, 0.1 ml of the diluted seed virus was injected into the chorioallantoic cavity. Molten paraffin (melting point: 50 to 52°C) was placed onto the hole using a Pasteur pipette in order to seal the hole. The egg was placed in an incubator and incubated for three days at 36. 5°C and 40% or more humidity. The inoculated egg was then left overnight at 4°C. The following day, the air cell portion of the egg was broken with forceps, and a 10 ml syringe with an 18 gauge needle was placed in the chorioallantois so as to aspirate the chorioallantoic fluid, which was collected in a sterilized bottle and stored at 4°C.

### (2: Purification of HVJ)

HVJ may be purified by purification methods utilizing centrifugation, purification methods utilizing a column, or any other purification methods known in the art.

### (2.1: Centrifugation-based purification method)

Briefly, a suspension of cultured viruses was collected, and the medium centrifuged at low speed to remove tissue or cell debris in the culture medium and the chorioallantoic fluid. The supernatant thereof was purified by high-speed centrifugation (27,500×g, 30 minutes) and ultracentrifugation (62,800×g,90 minutes) on a sucrose density gradient (30 to 60%w/v). Care should be taken to treat the virus as gently as possible during purification, and to store the virus at 4°C.

Specifically, in the present example, HVJ was purified by the following method.

About 100 ml of HVJ-containing chorioallantoic fluid (the chorioallantoic fluid from chicken eggs containing HVJ, which was collected and stored at 4°C) was placed in two 50 ml centrifuge tubes with a wide-mouth Komagome type pipette (see Saeki, Y., and Kaneda, Y: Protein modified liposomes (HVJ-liposomes) for the delivery of genes, oligonucleotides and proteins. Cell Biology; A laboratory handbook (2nd edition) ed. by J.E. Celis (Academic Press Inc., San Diego) vol. 4, 127 to 135,1998), centrifuged in a low-speed centrifuge at 3000 rpm and at 4°C for 10 minutes (without braking) to remove the tissue debris from the egg.

After centrifugation, the supernatant was dispensed into four 35 ml centrifuge tubes (designed for high-speed centrifugation), and centrifuged for 30 minutesinafixed-angle rotor at 27,000g, (with acceleration and braking). The supernatant was removed, BSS (10 mM Tris-HCl (pH 7.5), 137 mM NaCl, 5.4 mM KCl; autoclaved and stored at 4°C) (BSS is interchangeable with PBS) was added to the pellet in an amount of about 5 ml per tube, and allowed to stand at 4°C overnight. The following morning, the pellets were resuspended by gentle pipetting with a wide-mouth Komagome type pipette and collected in one tube, and then similarly centrifuged for 30 minutes in a fixed-angle rotor at 27,000g. The supernatant was removed, and about 10 ml of BSS was added to the pellet and allowed to stand at 4°C overnight. The following morning the pellets were resuspended by gentle pipetting with a wide-mouth Komagome type pipette and then centrifuged for 10 minutes in a low-speed centrifuge at 3000 rpm at 4°C (without braking), thereby removing tissue debris and agglutinated virus which had not been completely removed. The supernatant was placed in a fresh sterilized tube, and stored at 4°C as the purified virus stock.

To 0.1 ml of this virus solution, 0.9 ml of BSS was added, and the absorption at 540 nm was measured with a spectrophotometer. The virus titer was converted into an erythrocyte agglutination activity (HAU). An absorption value of 1 at 540 nm approximately corresponded to 15,000 HAU. It is considered that HAU is substantially proportional to fusion activity. Alternatively, erythrocyte agglutination activity may be measured by using a solution containing (0.5%) chicken erythrocytes (see DOUBUTSU SAIBO RIYO JITSUYOKA MANUAL (or "Practice Manual for Using Animal Cells"), REALIZE INC. (ed. by Uchida, Oishi, Furusawa) pp. 259 to 268, 1984).

Furthermore, purification of HVJ using a sucrose density gradient may be performed as necessary. Specifically, a virus suspension is placed in a centrifuge tube in which 60% and 30% sucrose solutions (autoclave-sterilized) are layered, and the density gradient centrifuged for 120 minutes at 62,800×g. After centrifugation, the virus is visible as a band at the interface of the 60% sucrose solution layer, and is recovered. The recovered virus suspension is dialyzed overnight at 4°C against an external solution of BSS or PBS, thereby removing the sucrose. In the case where the virus suspension is not to be immediately used, glycerol (autoclave-sterilized) and a 0.5 M EDTA solution (autoclave-sterilized) are added to the virus suspension so as to attain final concentrations of 10% and 2 to 10 mM, respectively, the suspension is then gently frozen at -80°C, and finally stored in liquid nitrogen (the frozen storage can be performed with 10 mM DMSO, instead of glycerol and a 0.5 M EDTA solution).

### (2.2: Purification method utilizing columns and ultrafiltration)

Instead of purification by centrifugation, purification of HVJ utilizing columns is also applicable to the present invention.

Briefly, concentration (about 10 times) via ultrafiltration utilizing a filter having a molecular weight cut-off (MWCO) of 50,000 and elution via ion exchange chromatography (0.3 M to 1 M NaCl) were performed to achieve purification.

Specifically, in the present example, the following method was used to purify HVJ.

After the chorioallantoic fluid was collected, the chorioallantoic fluid was filtrated through a membrane filter (80 µm to 10 µm). To the choricallantoic fluid, 0.006 to 0.008% BPL (final concentration) was added (4°C, 1 hour), so as to inactivate the HVJ. The chorioallantoic fluid was incubated for 2 hours at 37°C, thereby inactivating the BPL.

About 10 times concentration was achieved using a tangentialflow ultrafiltration method using a 500KMWCO membrane (A/G Technology, Needham, Massachusetts). As a buffer, 50 mM NaCl, 1 mM MgCl₂, 2% mannitol, and 20 mM Tris (pH 7.5) were used. An HAU assay indicated an HVJ yield of approximately 100%. Thus, excellent results were obtained.

HVJ was purified by column chromatography (buffer: 20 mM Tris HCl (pH 7.5), 0.2 to 1 M NaCl) using a Q Sepharose FF column (Amersham Pharmacia Biotech KK, Tokyo). The yield was 40 to 50%, and the purity was 99% or more.

An HVJ fraction was concentrated by tangential flow ultrafiltration using a 500KMWCO membrane(A/G Technology).

### (3: Inactivation of HVJ)

In the case where it was necessary to inactivate HVJ, this was performed by UV light irradiation or treatment with an alkylating agent, as described below.

### (3.1: UV light irradiation method)

One milliliter of HVJ suspension was placed in a dish having a diameter of 30 mm, and subjected to an irradiation at 99 or 198 mJ/cm². Although gamma-ray irradiation is also applicable (5 to 20 Gv), it does not provide complete inactivation.

### (3.2: Treatment with an alkylating agent)

Immediately before use, 0.01% β-propiolactone was prepared in 10 mM KH₂PO. The solution was kept at a low temperature during preparation, and the operation was quickly performed.

β-propiolactone was added to a final concentration of 0.01% to the HVJ suspension obtained immediately after purification, and the mixture was then incubated on ice for 60 minutes. Thereafter, the mixture was incubated at 37°C for 2 hours. The mixture was dispensed into Eppendorf tubes in 10,000 HAU aliquots, and centrifuged for 15 minutes at 15,000 rpm. The precipitate was stored at -20°C. Instead of using the aforementioned inactivation method, without storing the precipitate at -20°C, DNA may be incorporated into a vector by detergent treatment alone when constructing a vector.

### (4: Construction of an HVJ envelope vector)

To the HVJ which had been stored, 92 µl of a solution containing 200 to 800 µg of exogenous DNA was added, and well mixed by pipetting. This solution can be stored at -20°C for at least 3 months. By adding protamine sulfate to the DNA before mixing with HVJ, the expression efficiency was enhanced twofold or more.

This mixture was placed on ice for 1 minute, and 8 µl of (10%) octylglucoside was added. The tube was shaken on ice for 15 seconds, and allowed to stand on ice for 45 seconds. The treatment time with the detergent is preferably 1 to 5 minutes. Instead of octylglucoside, detergents such as Triton-X100(t-octylphenoxypolyethoxyethanol), CHAPS(3-[(3-cholamidopropyl)-dimethylammonio]-1-propane sulfonate), or NP-40 (nonylphenoxy polyethoxy ethanol) may also be used. The final concentrations of Triton-X100, NP-40, and CHAPS are preferably 0.24-0.80% (v/v), 0.04-0.12% (v/v) and 1.2-2.0% (v/v), respectively.

One milliliter of cold BSS was added, and the solution was immediately centrifuged for 15 minutes at 15,000 rpm. To the resultant precipitate, 300 µl of PBS or saline, etc., was added, and the precipitate suspended by vortexing or pipetting. The suspension may be directly used for gene transfer or may be used for gene transfer after storage at -20°C. After being stored for at least 2 months, this HVJ envelope vector maintained the same level of gene transfer efficiency.

### (Gene transfer method)

An amount of vector equivalent to 1, 000 HAU (30 µl) was placed into an Eppendorf tube, and 5 µl of protamine sulfate (1 mg/ml) was added. The medium was removed from BHK-21 cells (which were sown in 6 well dishes at a density of 200,000 cells per well the previous day) and 0.5 ml of medium (10%FCS-DMEM) was added to per well. To each well, a mixture of the aforementioned vector (equivalent to 1,000 HAU) and protamine sulfate was added, and the plate was shaken back and forth and from right to left, whereby the vector and cells were well mixed. The mixture was left in a 5%CO₂ incubator for 10 minutes at 37°C.

The medium was replaced, and the cells were left overnight (16 hrs to 24 hrs) at 37°C in a 5% CO₂ incubator, after which the gene expression was examined. To measure luciferase activity (pcLuci: a luciferase gene having a CMV promoter), the cells were lysed with 0.5 ml of Cell Lysis Buffer (Promega), and the activity in 20 µl of the solution was measured using a luciferase assay kit (Promega). To measure green fluorescent protein activity (pCMV-GFPE; Promega), the cells were observed under a fluorescent microscope in their intact form, and 5 to 8 fields were observed at a magnification of 400, and the ratio of cells which generated fluorescence was calculated.

### (Example 4: Preparation and use of a liposome vector)

### (Preparation of liposome vector)

A liposome vector of the present invention is prepared as follows: Twenty to twenty four µg of cDNA library-derived cDNA and 24-72 µL of lipofect AMINE 2000 reagent (In vitrogen life technologies (Carlsbad, California 92008) are respectively diluted in 1.2 mL of serum free medium, and rapidly mixed. The mixture is incubated at room temperature for 20 minutes, to form a complex of liposomes and nucleic acid.

### (Transfection by liposome vector)

Host cells are added to each well of 96-well plate together with an appropriate medium, and cultured. When transfection is conducted in the presence of serum, 12.5 µL of the transfection complex is directly added to each well of the 96-well plate and mixed. When transfection is conducted in absence of serum, the medium containing serum is removed and replaced by a serum free medium before adding the transfection complex. After incubation in a CO₂ incubator for 4 to 12 hours, the medium is replaced. After a predetermined period of culture, an assay is conducted.

### (Example 5: Isolation and analysis of a gene encoding vascular endothelial growth factor from a human heart cDNA library.)

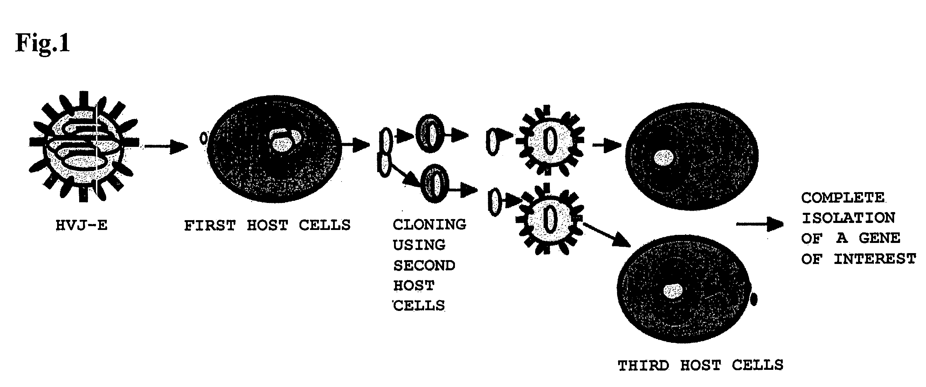

Using the present invention, it is possible to isolate a gene of interest having an intended functional property. One embodiment of the isolation method is schematically shown in Fig. 1.

Actually, using the gene transfer vector prepared in Example 3 of this specification, a gene encoding vascular endothelial growth factor was isolated. For isolating the gene exemplarily shown in this example, not only the gene transfer vector prepared in Example 3 of this specification, but also any "viral envelope vector" and "liposome vector" may be used.

Human heart cDNA library (GIBCO BRL; plasmid prepared by ligating human heart-derived cDNA to plasmid pSPORT having a CMV promoter) was transferred into E. coli DH12S, and the plasmid was prepared from the E. coli. Two hundred µg of plasmid was encapsulated in 10000 HAU of HVJ-E gene transfer vector (gene transfer vector prepared in Example 3 of the invention, 3 x 10⁹ particles). About 5000 human aortal endothelial cells (HAEC) (Sanko Junyaku) were added to each well of a 96-well micro titer plate together with growth medium and cultured overnight. The cultured cells were used as host cells. To each well containing host cells, 1/100 amount of the above HVJ-E was added. The wells were kept at 37°C for 30 minutes, and then the medium was replaced.

The medium used was a low nutrient condition medium having a serum concentration of 1%. Under these conditions, culture was conducted for one week. Under these conditions, growth of HAEC was not observed.

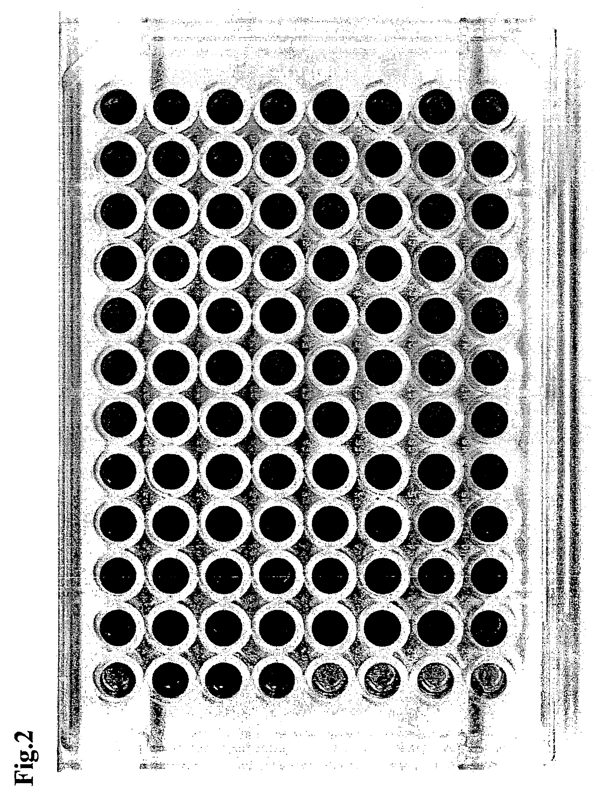

After two weeks, a cell growth assay was conducted. Using Cell Titer·96 (Promega) as a regent, cell growth was evaluated based on the color change that is indicative of the redox state of mitochondria. The result is shown in Fig. 2.

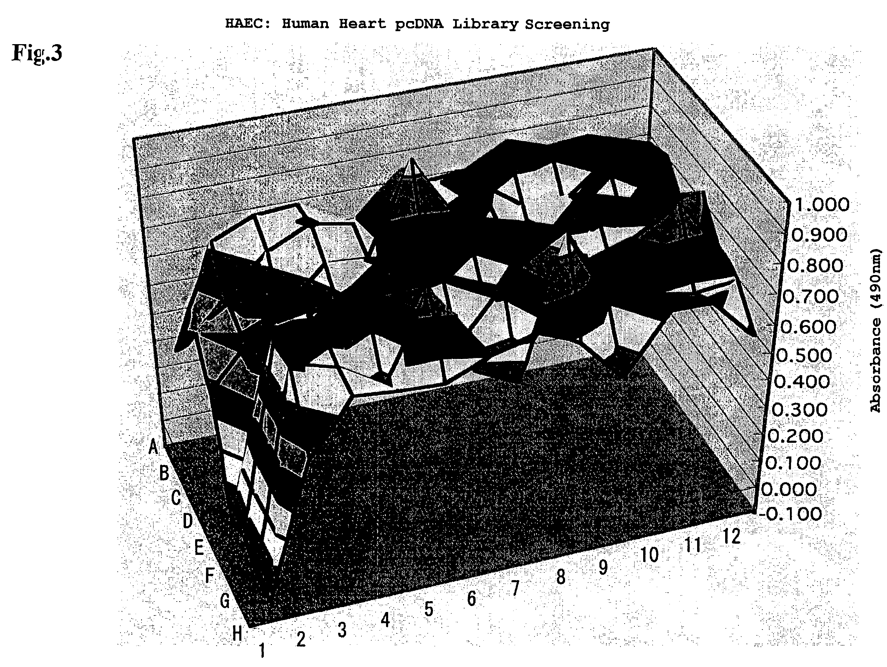

In Fig. 2, the wells having the deepest color are wells where cell growth occurred most actively. The entire micro titer plate was read with a plate reader, and cell growth was graphically shown with a computer as shown in Fig. 3. DNA was extracted from cells in the two wells exhibiting the greatest growth according to the graph, using a DNeasy Tissue Kit available from Qiagen. Since the prepared nucleic acid includes plasmid DNA, it was transferred into competent E. coli (DH5α; TAKARA) by heat shock.

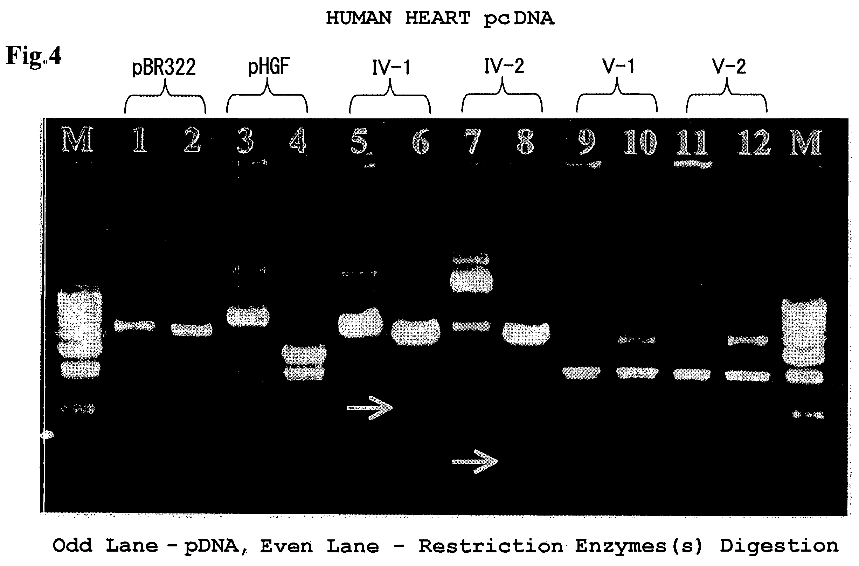

This E. coli was inoculated on an ampicillin-containing solid media, and allowed to form colonies. From DNA prepared from a single well, about 20-200 colonies were obtained. Plasmid DNA (pDNA) was extracted from each colony, and the presence of a gene fragment in the plasmid was confirmed by restriction enzyme analysis. About 60-70% of plasmids in the prepared plasmids were plasmids had an insert (the white arrow in Fig. 4 shows an insert fragment).

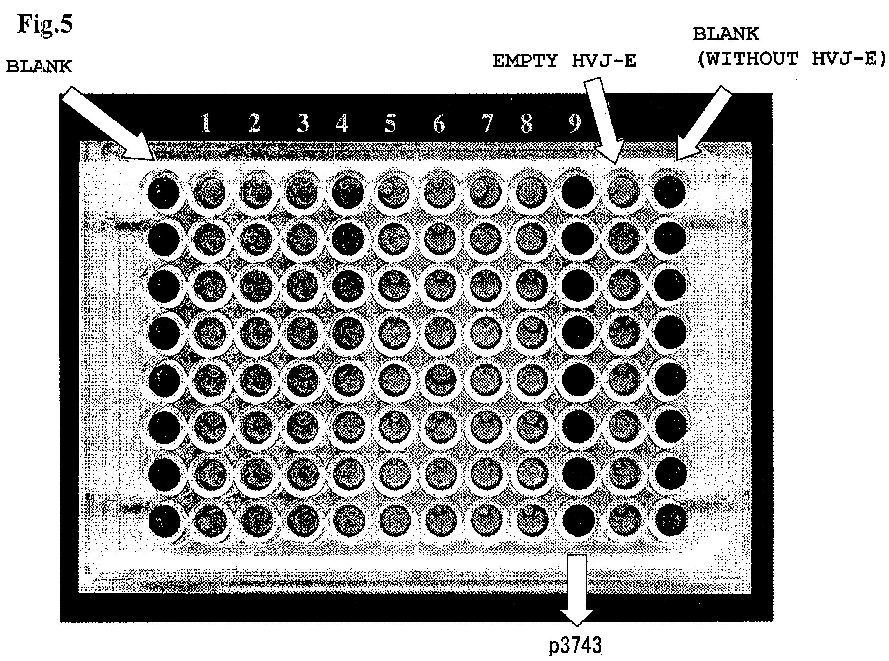

Next, plasmid DNA was purified using Endo Free Plasmid Maxi Kit available from Qiagen, and the purified plasmid was encapsulated in HVJ-E and transferred into HAEC cells again, and a cell growth test similar to that described above was conducted. In this cell growth test, the plasmid exhibiting significantly high cell growth is a candidate plasmid that is expected to include a nucleic acid encoding vascular endothelial growth factor. In the present example, two clones (p3743, p77421) exhibited high HAEC growth activity with high reproducibility. Results from one of these clones is shown in Fig. 5.

The gene products isolated in the present experiment had higher growth activity than VEGF or HGF with respect to human aortal endothelial cells HAEC. However, they exhibited similar activity with VEGF or HGF with respect to human vascular smooth muscle cells.

### (Discussion of angiogenetic activity)

The foregoing two genes exhibiting high HAEC growth activity (p3743, p77421) were evaluated for angiogenetic ability using an Angiogenesis Kit, KZ-1000 (KURABO INDUSTRIES LTD.) in accordance with the following procedure. As controls, blank, vascular endothelial growth factor-A protein (VEGF-A), pVEGF plasmid (a plasmid containing a gene encoding vascular endothelial growth factor) and pSPORT1 (a plasmid not containing a gene encoding vascular endothelial growth factor) were used. Each obtained image was quantified using an angiogenesis quantification software (KSW-5000U, KURABO INDUSTRIES LTD.) in accordance with the following procedure.

On a 24-well plate, an angiogenesis KIT KZ-1000 (KURABO INDUSTRIES LTD.) co-culture human umbilical vein endothelial cell and human adult skin-derived fibroblast was used. To a medium specified for angiogenesis (KZ-2400, KURABO INDUSTRIES LTD.) 10 ng/mL of VEGF-E (NZ-7) was added, and anti human VEGF-A neutralizing antibody was added in concentration of 0, 250, 500, 1000 ng/mL and the medium used to culture the above cells.

Anti-VEGF, Human, Mouse-Mono (26503.111) (R&D, Catalog No. MAB293) was used as the anti human VEGF-A neutralizing antibody. Culture was conducted at 37°C in a 5% CO₂ incubator. After 4, 7 and 9 days of culture, the medium was replaced with fresh media supplemented with the same additives. After 11 days of culture, the medium was removed, and staining was conducted using a lumen staining kit (for staining CD31 antibody: KURABO INDUSTRIES LTD. KZ-1225) in accordance with the following procedure.

CD31 (PECAM-1)-staining primary antibody (mouse anti-human CD31 antibody) was 4,000-fold diluted in blocking solution (Dulbecco phosphate buffer (PBS (-) containing 1% BSA). To each well, 0. 5 mL of this primary antibody solution was added, and incubated for 60 minutes at 37°C. After incubation, each well was washed a total of three times with 1 mL of blocking solution.

Then, 0.5 mL of a secondary antibody solution (goat anti-mouse IgG alkaline phosphatase complex) that was 500-fold diluted with a blocking solution was added to each well, incubated for 60 minutes at 37°C, and then washed three times with 1 mL of distilled water. During this, two tablets of BCIP/NBT were dissolved in 20 mL of distilled water, and filtered through a filter having a pore size of 0.2 µm, to prepare a substrate solution. Then 0.5 mL of the prepared BCIP/NBT solution was added to each well, and incubated at 37°C until the lumen turned deep violet (usually 5 to 10 minutes). After completion of incubation, each well was washed three times with 1 mL of distilled water, and the washing solution removed by aspiration. Then each well was left to stand in order to air dry. After drying, each well was observed under a microscope.

Each well was observed under x40 magnification, and photographed.

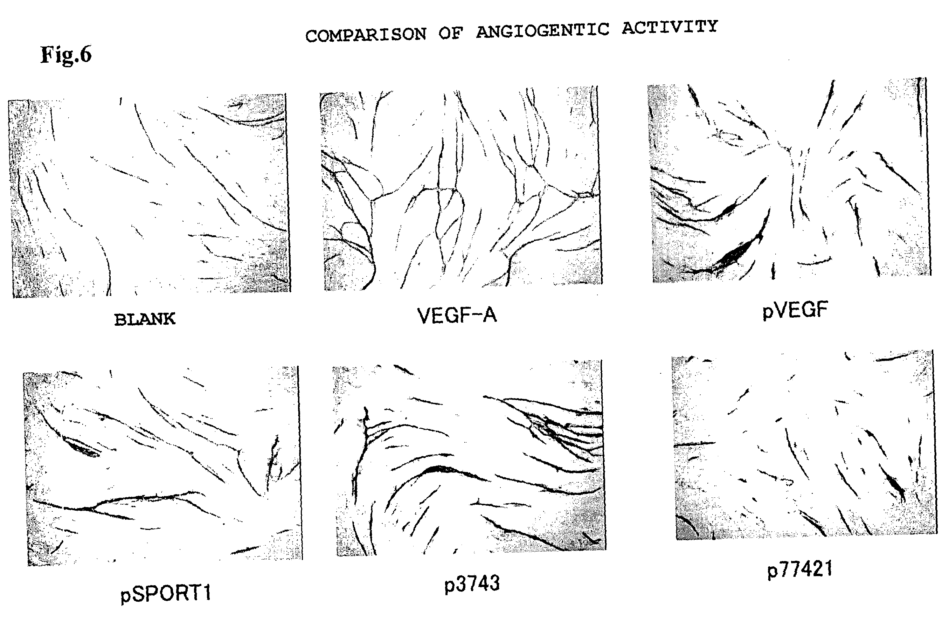

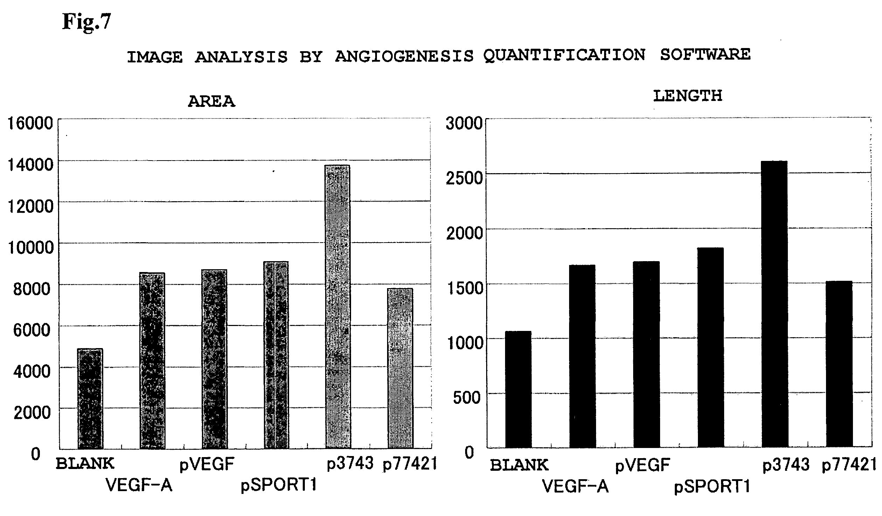

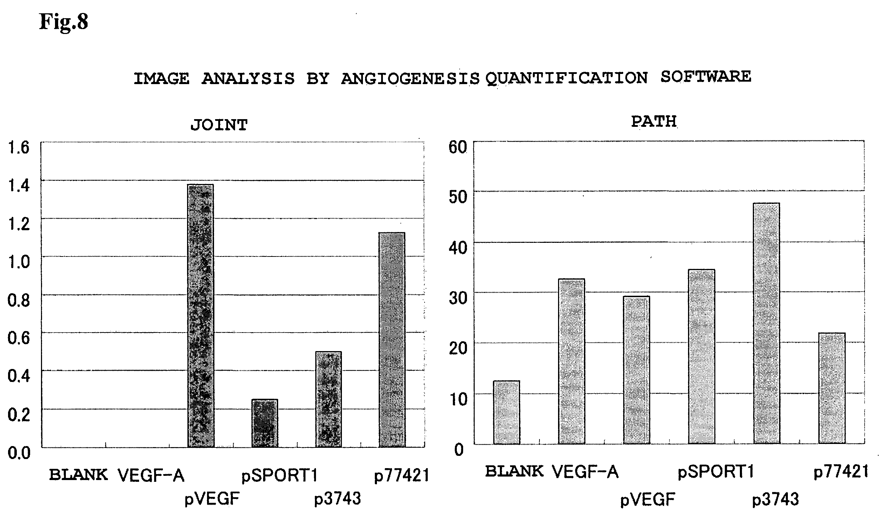

A picture in which a scale of 1 mm magnified 40-folds was taken (Fig. 6), and based on this scale, the area of the lumen (left in Fig. 7), the length of the lumen (right in Fig. 7), the joints of the lumen (left in Fig. 8) and the path of the lumen (right in Fig. 8) formed in each visual field were measured. The number of branch points of the lumen is denoted by "joint" and the number of lumens coming from the branch point is denoted by "path".

The observation result shown in Fig. 6 and the date of the lumen formed shown in Figs. 7 and 8 demonstrated that clone p77421 has a similar degree of angiogenetic activity to VEGF, and the clone p3743 has better angiogenetic activity than VEGF.

### (c-fos luciferase assay)

The influence that the product of the genes isolated in the foregoing experiment exerts on the activity of promoter of c-fos gene was examined by reporter assay using a reporter plasmid incorporating a c-fos gene promoter upstream of a luciferase gene.

Specifically, the assay was conducted in the following manner.

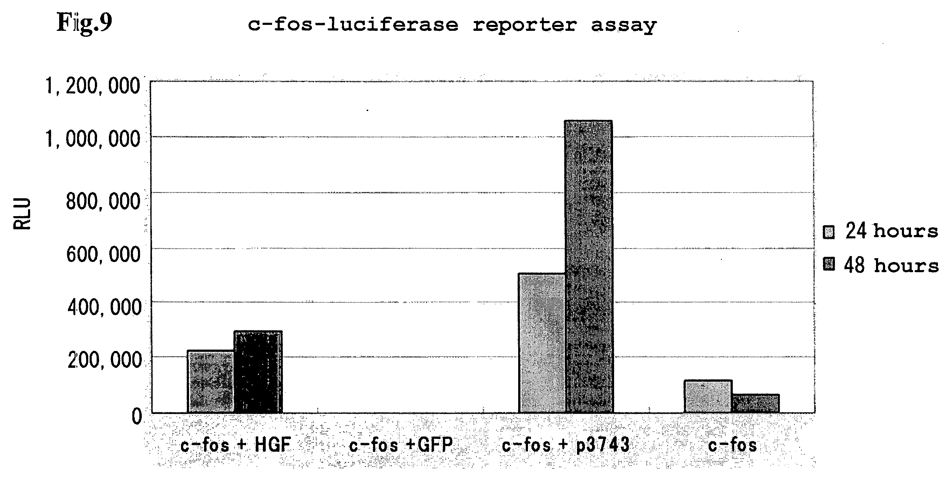

Endothelial cells were seeded in a 6-well plate, and transfected with a c-fos-luciferase reporter gene (p2FTL) using lipofect AMINE PLUS (GIBCO-BRL). This fos-luciferase reporter gene consists of 2 copies of the c-fos 5'-regulatory enhancer element (-357 to -276), the thymidine kinase gene promoter of herpes simplex virus (-200 to +70), and a luciferase gene. It was co-transfected with p3743 plasmid as necessary. Twenty four hours after transfection, transfected cells were incubated in a serum free medium for 24 hours. As necessary, cells in rest state were treated with 100 ng/ml of HGF (hepatocyte growth factor) or with GFP (green fluorescent protein) for four hours. After washing with PBS and adding with 500 µL of cell lysis buffer, the cells were kept at room temperature for 15 minutes, and thereby lysed. 10 µL of cell extract obtained by lysing cells was mixed with 100 µL of luciferase assay reagent, and light emission was measured for 30 seconds using a luminometor in units of RLU. The significant increase in luciferase activity demonstrated that p3743 increases c-fos gene promoter activity (Fig. 9).

The above result demonstrates that a gene having an intended functional property may be readily and conveniently isolated by using the present invention.

### (Example 6: Isolation method of mutant nucleic acid having an intended functional property)

With the present invention, it is possible to isolate a mutant gene having an intended functional property.

Using a gene transfer vector prepared in Example 3 of this specification, a gene encoding vascular endothelial growth factor is isolated. For isolating the gene exemplarily shown in this example, not only the gene transfer vector prepared in Example 3 of this specification, but also any "viral envelope vector" and "liposome vector" may be used.

As a starting material, a nucleic acid comprising a specified gene is selected. A plasmid in which the selected nucleic acid is operably linked to a sequence that functions as a promoter in a first host cell is constructed. The plasmid is transferred into a first host cell according to Example 3.

The first host cell into which the nucleic acid is transferred is subjected to mutagenesis, and about 5000 cells are added on each well of 96-well micro titer plate together with a medium, followed by overnight cultivation. After cultivation, mutated host cells in each well are screened for an intended function. Cells having an intended function are isolated, and nucleic acid is extracted from the cells in the well exhibiting the most desired property using a DNeasy Tissue Kit available from Qiagen. Since the prepared nucleic acid includes plasmid DNA, it is transferred into competent E. coli (DH5α; TAKARA) by heat shock transformation.

This E. coli is inoculated onto ampicillin-containing solid media, and allowed to form colonies. From DNA prepared from a single well, about 20-200 colonies can be obtained. Plasmid DNA is extracted from each colony, and the presence of a gene fragment in the plasmid is confirmed by restriction enzyme digestion.

Then, using an Endo Free Plasmid Maxi Kit available from Qiagen, plasmid DNA is purified, and the purified plasmid is encapsulated in HVJ-E and transferred into HAEC cells again, and a similar functional property is examined. In this cell growth test, the plasmid exhibiting a preferred functional property is recognized as a plasmid containing a mutant nucleic acid having an intended functional property.

Although the present invention has been described in its preferred embodiments, it should be noted that the present invention is not limited to these embodiments. Thus, it can be understood that the scope of the present invention is defined only by the claims attached hereto. It can be understood that those skilled in the art can practice an equivalent scope based on the description of the present invention and the common general knowledge in the art in view of the description of the specific preferred embodiments. It is appreciated that patents, patent applications and documents cited in this specification are incorporated herein by reference as if the content thereof is specifically described in the present specification.

### Industrial Applicability

A novel method and a kit for isolating a nucleic acid having an intended functional property are provided. As a result, it is possible to isolate a nucleic acid having an intended functional property more rapidly and conveniently than the conventional method. The present invention is particularly effective for expression screening using mammalian cells as a host cell.

In the present invention, by changing the combination of an objective cell and an assay method, it is possible to isolate genes having a variety of different functions. Specific examples of such genes include tumor-suppressor genes; osteogenesis enhancer genes; apoptosis trigger genes; cytokine secretion genes; nerve cell dendrite inducer genes; arteriosclerosis suppressor genes; diabetes suppressor genes; autoimmune diseases suppressor genes; Alzheimer's disease suppressor genes; Parkinson's disease suppressor genes and nerve cell protecting genes and the like.

## Claims

1. A method of isolating a nucleic acid having an intended functional property, comprising the steps of:
(A) transferring a nucleic acid into a plurality of first host cells and allowing the nucleic acid to transiently express therein;
(B) selecting, from the plurality of first host cells into which the nucleic acid is transferred, a cell into which a nucleic acid having an intended functional property has been transferred;
(C) preparing a purified nucleic acid from the selected cell; and
(D) selecting a purified nucleic acid having an intended functional property.

2. The method according to claim 1, wherein at least two kinds of nucleic acids are transferred into the plurality of first host cells.

3. The method according to claim 1, wherein the step of transferring a nucleic acid into the plurality of first host cells is carried out according to a procedure selected from the group consisting of: a transferring method using a viral envelope, a transferring method using a liposome, a transferring method using a liposome containing at least one protein from a viral envelope, a transferring method using calcium phosphate and an electroporation method.

4. The method according to claim 1, wherein the nucleic acid includes a foreign gene and a promoter.

5. The method according to claim 1, wherein the host cells are mammalian cells.

6. The method according to claim 1, wherein the host cells are human cells.

7. The method according to claim 1, wherein the viral envelope is derived from wild-type or recombinant viruses.

8. The method according to claim 1, wherein the viral envelope is derived from a virus belonging to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae.

9. The method according to claim 8, wherein the virus is derived from viruses belonging to the family Paramyxoviridae.

10. The method according to claim 9, wherein the virus is HVJ.

11. The method according to claim 1, wherein the vector is a viral envelope vector.

12. The method according to claim 1, wherein the vector is a vector containing a protein prepared from a viral envelope and a liposome.

13. The method according to claim 12, wherein the protein prepared from a viral envelope is a protein selected from the group consisting of F protein, HN protein, NP protein and a combination thereof.

14. The method according to claim 1, wherein the step (C) of preparing a purified nucleic acid from the selected cell is carried out in the following steps of:
(i) extracting a nucleic acid from the selected cell;
(ii) transferring the extracted nucleic acid into a second host cell to thereby obtain a transformed cell;
(iii) purifying the transformed cell; and
(iv) preparing a nucleic acid from the purified transformed cell.

15. The method according to claim 14, wherein the second host cell is a bacterium or fungus.

16. The method according to claim 15, wherein the nucleic acid contains a sequence that is necessary for autonomous replication in the bacterium or fungus.

17. The method according to claim 15, wherein the bacterium belongs to a genus selected from the group consisting of Escherichia, Bacillus, Streptococcus, Staphylococcus, Haemophilus, Neisseria, Actinobacillus and Acinetobacter.

18. The method according to claim 17, wherein the bacterium is Escherichia coli.

19. The method according to claim 15, wherein the fungus is Saccharomyces, Schizosaccharomyces or Neurospora.

20. The method according to claim 1, wherein the step (D) of selecting a purified nucleic acid having an intended functional property is carried out in the following steps of:
(i) transferring the purified nucleic acid into a third host cell to obtain a transformed cell;
(ii) comparing the property of the transformed cell with the property of a third host cell that is not transformed; and
(iii) determining whether or not the transformed cell has an intended functional property, as a result of the comparison.

21. The method according to claim 20, wherein the step (D) of selecting a purified nucleic acid having an intended functional property further includes the step of (iv) preparing a nucleic acid having an intended functional property from the selected cell.

22. The method according to claim 20, wherein the third host cell is a mammalian cell.

23. The method according to claim 20, wherein the third host cell is a human cell.

24. The method according to claim 20, wherein the third host cell is derived from the same species as the species from which the first host cell is derived.

25. The method according to claim 1, wherein the intended functional property is selected from the group consisting of induction of angiogenesis, tumor suppression, enhancement of osteogenesis, induction of apoptosis, cytokine secretion, induction of dendrites, suppression of arteriosclerosis, suppression of diabetes; suppression of autoimmune diseases; suppression of Alzheimer's disease, suppression of Parkinson's disease, protection of nerve cells and combinations thereof

26. A kit for isolating a nucleic acid having an intended functional property, comprising:
(A) a nucleic acid transfer vector to be transferred into a plurality of first host cells in order to transform said first host cells; and
(B) a second host cell for preparing a purified nucleic acid from a cell selected from the transformed first host cells.

27. The kit according to claim 26, wherein the nucleic acid transfer vector is a viral envelope, liposome or liposome containing at least one protein from a viral envelope.

28. The kit according to the claim 26, wherein the first host cells are mammalian cells.

29. The kit according to claim 26, wherein the first host cells are human cells.

30. The kit according to claim 26, wherein the viral envelope is derived from wild-type or recombinant viruses.

31. The kit according to the 26, wherein the viral envelope is derived from a virus belonging to a family selected from the group consisting of Retroviridae, Togaviridae, Coronaviridae, Flaviviridae, Paramyxoviridae, Orthomyxoviridae, Bunyaviridae, Rhabdoviridae, Poxviridae, Herpesviridae, Baculoviridae and Hepadnaviridae.

32. The kit according to claim 26, wherein the virus is derived from viruses belonging to the family Paramyxoviridae.

33. The kit according to claim 26, wherein the virus is HVJ.

34. The kit according to claim 26, wherein the vector is a viral envelope vector.

35. The kit according to claim 26, wherein the vector is a vector containing a protein prepared from a viral envelope and a liposome.

36. The kit according to claim 35, wherein the protein prepared from viral envelope is a protein selected from the group consisting of F protein, HN protein, NP protein and a combination thereof.

37. The kit according to claim 26, wherein the second host cell is a bacterium or fungus.

38. The kit according to claim 26, further comprising a nucleic acid for preparing a nucleic acid to be transferred into the first host cells.

39. The kit according to claim 26, further comprising a reagent to be used for determining whether or not the purified nucleic acid has an intended functional property.

40. The kit according to claim 37, wherein the bacterium belongs to a genus selected from the group consisting of Escherichia, Bacillus, Streptococcus, Staphylococcus, Haemophilus, Neisseria, Actinobacillus and Acinetobacter.

41. The kit according to claim 40, wherein the bacterium is Escherichia coli.

42. The kit according to claim 37, wherein the fungus is Saccharomyces, Schizosaccharomyces or Neurospora.

43. A nucleic acid isolated by the method according to claim 1.

44. Use of a viral envelope for isolating a nucleic acid having an intended functional property.

45. Use of a liposome containing at least one protein from a viral envelope, for isolating a nucleic acid having an intended functional property.
